(19)

Europäisches
Patentamt
European
Patent Office
Office européen
des brevets

(11) **EP 4 776 446 A2**

(12) **EUROPEAN PATENT APPLICATION**

(43) Date of publication:
**15.07.2026 Bulletin 2026/29**

(21) Application number: **26181711.8**

(22) Date of filing: **02.07.2020**

(51) International Patent Classification (IPC):
***H01S 3/10*** *(2006.01)*

(52) Cooperative Patent Classification (CPC):
**A24F 40/465; A24F 40/42; A24F 40/51;**
**A24F 40/57; A61M 11/042; A61M 15/002;**
**A61M 15/0048; A61M 15/0066; A61M 15/0086;**
**A61M 15/06;** A61M 2016/0024; A61M 2016/0027;
A61M 2205/0227; A61M 2205/273;
A61M 2205/3646; (Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **26.03.2020 US 202063000456 P**

(62) Document number(s) of the earlier application(s) in
accordance with Art. 76 EPC:
**20926819.2 / 4 117 473**

(71) Applicant: **CQENS TECHNOLOGIES INC.**
**Minneapolis, MN 55419 (US)**

(72) Inventors:
• **CHONG, Alexander Chinhak**
**Minneapolis, MN, 55419 (US)**
• **BARTKOWSKI, William**
**Minneapolis, MN, 55419 (US)**

• **CROSBY, David**
**Minneapolis, MN, 55419 (US)**
• **WAYNE, David**
**Minneapolis, MN, 55419 (US)**
• **SHUDALL, Gerard**
**Minneapolis, MN, 55419 (US)**

(74) Representative: **Prüfer & Partner mbB**
**Patentanwälte · Rechtsanwälte**
**Sohnckestraße 12**
**81479 München (DE)**

Remarks:
This application was filed on 28-05-2026 as a
divisional application to the application mentioned
under INID code 62.

(54) **HEAT-NOT-BURN DEVICE AND METHOD**

(57) A method for aerosolizing a consumable by an aerosol producing device. The method comprises preheating a susceptor surrounded by the consumable to a moderate temperature that is above room temperature, but below an aerosolizing temperature, and heating the susceptor from the moderate temperature to the aerosolizing temperature to aerosolize the consumable. The amount of power needed to heat the susceptor to the aerosolizing temperature may be calculated by extrapolating a temperature drop over time. Preheating may occur upon insertion of a consumable-containing package into the device or by actuation of an external actuator. Heating to the aerosolizing temperature may be triggered when a pressure sensor detects a pressure drop inside the device. After aerosolizing, the susceptor may be brought back down to the moderate temperature or an intermediate temperature. The preheating and heating may be performed by a heating element, such as a coil wrapped around the susceptor.

Fig. 1

(52) Cooperative Patent Classification (CPC): (Cont.)
A61M 2205/368; A61M 2205/505; A61M 2205/52;
A61M 2205/584; A61M 2205/587;
A61M 2205/6018; A61M 2205/6054;
A61M 2205/6072; A61M 2205/6081;
A61M 2205/75

**Description**

TECHNICAL FIELD

This invention relates to methods, formulations, and devices for aerosolizing medicants via a high-temperature, non-combusting inductive heating method, and uses thereof. The invention further relates to methods and devices for producing an aerosol from tobacco and/or other non-medicant substances using similar methods.

BACKGROUND

When faced with a condition giving rise to bodily discomfort, such as a diseased state, disorder, ailment, normal bodily disruptions, and the like, most people turn to medicants, such as drugs, supplements, herbs, and the like for immediate relief from the symptoms that arise from the underlying condition. There are certain legal and widely available over-the-counter (OTC) medications and supplements that have beneficial effects when used for a variety of common conditions. There are also certain controlled narcotics and pharmaceuticals prescribed by doctors for a variety of more serious conditions.

One of the most common routes of administration of these OTC and prescription drugs is oral administration. As with any oral delivery of medication, however, it must pass through the digestive tract. There are a number of disadvantages of oral administration. For example, because the drug has to pass through the digestive system, the onset of activation of the drug is slow. In addition, in the digestive tract the drug may be inactivated or destroyed, and therefore, lose its potency or efficacy. The drug itself can also cause problems in the digestive tract, or side effects, such as loss of appetite, diarrhea, acidity, and the like. Furthermore, patients may be reluctant or unable to swallow oral medication in the form of a pill.

Certain medicants are intended to affect the brain or the brain's actions or activities but, given the accepted method of ingestion--gastrointestinal, intravenous, or intramuscular--these medicants can also have a variety of discomforting side effects due to the nature of ingestion or injection. These include, but are not limited to gastro-intestinal complications, digestive disorders, high blood pressure, and/or headaches, as well as the reluctance of users to self-administer medicants by injection.

Other routes of delivery exist, such as intradermal injections, patch applications, inhalations, and the like. Each of these has its own advantages and disadvantages. Therefore, there is still room for improving routes of administration of medicants.

For example, there are varieties of medicants that are safer, more effective, and more efficient with respect to both safety and efficacy if their ingestion is via inhalation of an aerosol, such as a gas, vapor, mist, and any other inhalant, containing the medicant or its active ingredient rather than by gastrointestinal, intravenous or intramuscular delivery.

Additionally, certain methods to aerosolize and deliver these medicants have drawbacks as well, specifically those that aerosolize the medicant itself, changing the molecular or chemical structure of the medicant or those that might aerosolize at a high temperature-extending the duration heating and raising the risk of changing the molecular or chemical structure of the active ingredient. Other drawbacks of current aerosolization techniques include the transport, storage and merchandising of certain of these medicants in cartridges that are prone to leaking and, in many cases, are designed and constructed with cartridge materials that are not environmentally friendly, containing plastics and other materials that are not biodegradable.

In order to ensure that the medicant is delivered intact via the high temperature, non-combusting inductive method, it is preferred that the method of aerosolization does not change the chemical or fundamental molecular structure of the medicant or other materials that make up the medicant, or if such changes occur, that they will not interfere with, and/or improve, the efficacy of the medicant.

Therefore, there is still a need for improving the routes of administration of medicants. In particular, there is still a need for improving methods of aerosolizing medicants for inhalation that would also provide the added benefit of metering, monitoring and measuring inhalers exact dosages without destroying the active ingredient or adding other chemicals to the aerosol as a result of energy inefficiency or prolonged heating duration. There is also the need for consumable embodiments that are biodegradable and do not contain materials that are not consistent with environmentally friendly disposal.

In addition to medicant delivery systems, heat-not-burn (HNB) devices are a type of device generally used to heat tobacco at temperatures lower than those that cause combustion to create an aerosol containing nicotine and other tobacco constituents, which is then made available to the device's user. In some embodiments, the heated element or susceptor is placed inside a solid tobacco product with a coil wrapped around the tobacco product and susceptor to cause the susceptor to heat through an inductive mechanism. Unlike traditional cigarettes, the goal is not to burn the tobacco, but rather to heat the tobacco sufficiently to release the nicotine and other constituents through the production of aerosol. Igniting and burning the cigarette creates unwanted toxins that can be avoided using the HNB device. There is a fine balance, however, between providing sufficient heat to effectively release the tobacco constituents in aerosol form and not burn or ignite the tobacco. Current HNB devices on the market have not found that balance, either heating the tobacco at temperatures that produce an inadequate amount of aerosol or over heating the tobacco and producing an unpleasant or "burnt" flavor profile. Additionally, the current methodology leaves traditional HNB device internal components dirtied with burning tobacco byproducts and the byproducts of accidental combustion.

Furthermore, in order to ensure the state change from a solid or liquid state to an aerosol state in a rapid, energy efficient manner via high temperature, non-combusting inductive heating, the formulation must be configured in a way that eliminates air flow between the formulation and the inductive system's susceptor.

For the foregoing reasons there is a need for a device, method, and formulation that provides its user the ability to control the power of the device, which will affect the temperature at which the tobacco will be heated via the inductive method to reduce the risk of combustion - even at what would otherwise be sufficient temperatures to ignite - while increasing the efficiency and flavor profile of the aerosol produced.

SUMMARY

The present invention is directed towards devices, methods, and formulation for delivering a consumable in an aerosolized state for inhaled administration and ingestion using a high temperature, non-combusting inductive method to aerosolize an embodiment of the formulation's design and configuration.

In particular, the present invention is directed towards further improvements in heat-not-burn devices, such as that described in U.S. Provisional Application No. 63/000,456 filed March 26, 2020 which application is incorporated in its entirety here by this reference. In general, the heat-not-burn device is a device for converting a consumable into an aerosol that contains certain of its constituents but limiting the byproducts most often associated with combustion, for example, smoke, ash, tar and certain other potentially harmful chemicals. It does so by using high heat without burning the consumable by packaging the consumable containing an internal susceptor inside an encasement. This invention can involve positioning and incrementally advancing heat along a consumable tobacco component with the use of an induction heating element wrapped around the consumable-containing package to heat the susceptor using a magnetic field generated by the induction heating element.

An object of the present invention is a device wherein an induction heating source is provided for use to heat a consumable tobacco component.

Another object of the present invention is a consumable tobacco component comprised of several, sealed, individual, airtight, coated encasements containing a consumable tobacco preparation - and an induction heating source. The encasement may be an aluminum shell with pre-set openings. The encasements may be coated with a gel that seals the openings until an inductive heating process melts the gel, clearing the openings. In some embodiments, the gel can include a flavoring agent that can add flavor to or enhance the flavor of the tobacco aerosol.

In some embodiments, multiple encasements are stacked inside a paper tube with spaces between them, formed by excess aluminum wrapping at the bottom end of each encasement and channels on either side to allow for the aerosol produced. When the inductive heating source is activated, the pre-set openings are cleared, and flavor is combined with the aerosol to travel through the tube and be made available to the user of the device.

Using these methods and apparatus, the device is required to heat less mass, can heat-up immediately, cool down quickly and conserve power, allowing for greater use between recharging sessions. This contrasts with the well-known, current, commercially available heat-not-burn devices.

Another object of the present invention is a tobacco-containing consumable component comprised of several, sealed, individual, airtight, coated encasements and an induction heating source. The encasements are then coated with a gel that seals them until an inductive heating process can melt the gel, clearing the openings. In some embodiments, the gel can include a flavoring agent that can add flavor to or enhance the flavor of the consumable tobacco component.

Another object of the present invention is to create a consumable-containing package that is easy to replace and minimizes fouling the inside of the case during use so as to reduce cleaning efforts of the case.

Another object of the present invention is to move the heating element relative to the susceptor or the consumable to heat segments of the consumable independent of other segments.

Another object of the invention is to maximize the efficiency of energy usage in the device for generating aerosol.

Another object of the invention is to aerosolize the consumable, which can be compressed around a susceptor in such a way as to eliminate any flow of air between the consumable and the susceptor. For example, the consumable-containing unit can contain inert non-reactive compounds that are mixed with a form of the consumable and then tightly compressed around a susceptor. The formulation can be aerosolized using a hand-held high temperature inductive heating device configured to the embodiment of the consumable.

The present invention further improves on the heat-not-burn device by utilizing a paper-thin susceptor made of a metallic wool that heats efficiently and is manufactured easily, thereby saving on costs.

In some embodiments, the efficiency is improved with an easy-to-apply encasement.

In some embodiments, the efficiency is improved with the use of a valve to control the pressure differential created inside the consumable-containing package.

In some embodiments, the efficiency of the device is improved with a unique seal configuration to seal the space between a consumable-containing package and a receiver in a high temperature, non-combusting inductive method designed to aerosolize the consumables contained and configured in an embodiment of the consumable without causing combustion to take place.

In some embodiments, the efficiency of the device is improved with a recognition system. A recognition system can identify specific characteristics and features of a consumable and communicate with the system controller to control how the consumable is administered based on the profile of the consumable. For example, the consumable can be treated with a marker, such as an ink or dye (visible or invisible), that can be read by a sensor in the device. The characteristics of the marker, once determined by the sensor, can allow the device to recognize the various characteristics that the device has been programmed to recognize, and adjust the temperature, duration of the heating, and the number of doses to best heat the consumable consistent with its flavoring and enhancing the consumer experience. Additionally, the recognition system can cause the color of the consumable to change after it has been used for a specifically programmed number of puffs, indicating when the consumable has been fully consumed. Such an indication would then render the consumable unable to be used in the device any longer. Finally, the recognition system can be used to trigger a disabling response to render the consumable unusable. For example, a burst of power can be produced that causes a hole in the consumable that renders it unable to be used any longer.

Accordingly, the device, method, and formulation of the present invention can be used to aerosolize a variety of consumables, preferably, medicants. For example, these medicants include, but are not limited to those configured to increase bronchial efficiency, support tobacco and nicotine cessation, assist in relaxation, ease anxiety, discourage disruptive ideation, manage pain, increase concentration, aid in restful sleep, aid in sexual activity, increase energy and wakefulness and counteract the harmful effects of the overdosing of certain other medicants. In addition, these consumables may include tobacco, cannabis, or other substances that can be ingested via inhalation by consumers.

BRIEF DESCRIPTION OF DRAWINGS

Figure 1 shows a side view inside of an embodiment of the present invention.

Figure 2A shows a perspective view of an embodiment of the present invention with portions removed to show inside the embodiment.

Figure 2B shows a perspective view of the embodiment shown in Figure 2A with portions cut away and/or removed to reveal internal components.

Figure 2C shows a cross-sectional view of the embodiment shown in Figure 2A cut along line 2C-2C.

Figure 2D shows an exploded view of the embodiment shown in Figure 2A.

Figure 2E shows a perspective view of another embodiment of the present invention with portions cut away and/or removed to reveal internal components.

Figure 2F shows a perspective view of another embodiment of the present invention with portions cut away and/or removed to reveal internal components.

Figure 3A shows a perspective view of another embodiment of the present invention.

Figure 3B shows a partially exploded view of the embodiment shown in Figure 3A.

Figure 3C shows a perspective view of the embodiment shown in Figure 3A with portions cut away and/or removed to reveal internal components.

Figure 3D shows a close-up, perspective view of a consumable-containing unit shown in Figure 3A.

Figures 4A and 4B show an exploded views of embodiments of a consumable-containing package.

Figure 4C shows an perspective view of another embodiment of the consumable-containing package with portions cut away or removed to reveal internal components.

Figure 4D shows a cross-sectional view of an embodiment of a consumable-containing package.

Figure 4E shows an perspective view of another embodiment of the consumable-containing package with portions cut away or removed to reveal internal components.

Figure 5A shows a perspective view of another embodiment of the present invention.

Figure 5B shows a cross-sectional view of the embodiment shown in Figure 5A taken along line 5B-5B.

Figure 5C shows a perspective view of a consumable-containing package from the embodiment shown in Figure 5A.

Figure 6A shows a perspective view of another embodiment of the present invention.

Figure 6B shows an exploded view of the embodiment shown in Figure 6A.

Figures 7A and 7B show perspective views of other embodiments of the present invention.

Figure 7C shows another embodiment of the consumable-containing package.

Figure 7D shows an exploded view of the embodiment shown in Figure 7C.

Figure 8A shows a side view of an embodiment of the heating element.

Figure 8B shows a front view of the heating element shown in Figure 8A.

Figure 9A shows a side view of an embodiment of the aerosol producing device.

Figure 9B shows a top view of the aerosol producing device.

Figure 9C shows a schematic diagram of an embodiment of the controller and its connection to other components of the present invention.

Figures 10A-10B show schematic diagrams of embodiments of the controller and its connection to other components of the present invention.

Figure 11A shows a perspective view of an embodiment of a moveable heating element with a recognition system.

Figure 11B-11E show another embodiment of the recognition system.

Figures 12A-12D show exploded views, cross-sectional views and perspective views of an embodiment of the present invention using a magnet for alignment.

Figure 12E shows a perspective view of another embodiment of an alignment mechanism.

Figures 13A-13B show perspective views of a multi-pronged susceptor.

Figures 13C-D show cross-sectional side views of the embodiments in Figures 13A and 13B, respectively, cut along the longitudinal axis showing the multi-pronged susceptor removed and inserted into the consumable-containing package.

Figures 14A-14C show end views of an embodiment of the consumable-containing package with the heating element rotating about the consumable-containing package.

Figures 15A-15C show end views of an embodiment of the consumable-containing package having another three-pronged susceptor with the heating element rotating about the consumable-containing package.

Figures 16A-16D show end views of an embodiment of the consumable-containing package having a four-pronged susceptor with the heating element rotating about the consumable-containing package.

Figures 17A-17B show perspective views of an embodiment of a mechanism for rotating the heating element along an eccentric path about the consumable-containing package.

Figures 18A-18B show end views of the embodiment in Figures 17A-17B of a mechanism for rotating the heating element along an eccentric path about the consumable-containing package.

Figure 19 shows a perspective view of an embodiment of a mechanism for rotating the heating element along an eccentric path and translating the heating element along the consumable-containing package.

Figure 20 shows a perspective view of an embodiment of a mechanism for moving the heating element relative to the consumable-containing package.

Figure 21 shows a schematic diagram of an embodiment of the controller and its connection to other components of the present invention.

Figure 22 shows an embodiment of a heat sink attached to the heating element, with portions of the heat sink removed to show the heating element.

Figure 23A shows a cross-sectional view of an airflow controller attached to the consumable-containing package.

Figure 23B shows another embodiment of the airflow controller attached to a receiver.

Figure 23C-23J show various embodiments of seals.

Figure 24A shows an exploded perspective view of another embodiment of the present invention.

Figure 24B shows an end view of the embodiment in Figure 24A.

Figure 24C shows a cross-sectional view taken through line 24C-24C shown in Figure 24B.

Figures 25A-B show partial cutaway views of the consumable-containing package in perspective with the susceptor removed to show a configuration inside the consumable-containing package that uses a hollow-pronged susceptor.

Figures 25C-D show partial cutaway views of the embodiments in Figures 25A-B, respectively, with the hollow-pronged susceptor embedded into a consumable-containing package.

Figure 25E shows a cross-sectional view of the embodiment shown in Figures 25A-D cut along its longitudinal axis to show the air flow during use.

Figure 26A shows a perspective view of another embodiment of the consumable-containing package prior to insertion of a susceptor.

Figures 26B-C show partial cutaway views of the embodiment shown in Figure 26A to show the relationship of the internal components prior to insertion of the susceptor.

Figure 26D shows a cross-sectional view of the embodiment of the consumable-containing package shown in Figures 26A-C cut along it longitudinal axis.

Figure 26E shows a partial cutaway view of the embodiment shown in Figure 26A after insertion of the susceptor.

Figure 26F shows the partial cutaway view shown in Figure 26E with a heating element wrapped around the consumable-containing package.

Figure 26G shows a cross-sectional view of the embodiment of the consumable-containing package shown in Figures 26F cut along it longitudinal axis.

DETAILED DESCRIPTION OF THE INVENTION

The detailed description set forth below in connection with the appended drawings is intended as a description of presently-preferred embodiments of the invention and is not intended to represent the only forms in which the present invention may be constructed or utilized. The description sets forth the functions and the sequence of steps for constructing and operating the invention in connection with the illustrated embodiments. It is to be understood, however, that the same or equivalent functions and sequences may be accomplished by different embodiments that are also intended to be encompassed within the spirit and scope of the invention.

The invention of the present application is a device for generating aerosols from a consumable-containing product for inhalation in a manner that utilizes relatively high heat with minimal burning of the consumable-containing product. For the purposes of this application, the term "consumable" is to be interpreted broadly to encompass any type of pharmaceutical agent, drug, chemical compound, active agent, constituent, any other medicant, and the like, regardless of whether the consumable is used to treat a condition or disease, is for nutrition, is a supplement, or used for recreation. By way of example only, a consumable can include pharmaceuticals, nutritional supplements, and over-the-counter medicants, such as but not limited to, tobacco, cannabis, hemp, lavender, kava, coffee, caffeine, lobelia, hoodia, melatonin, epedimium, guarana, ginseng and the like.

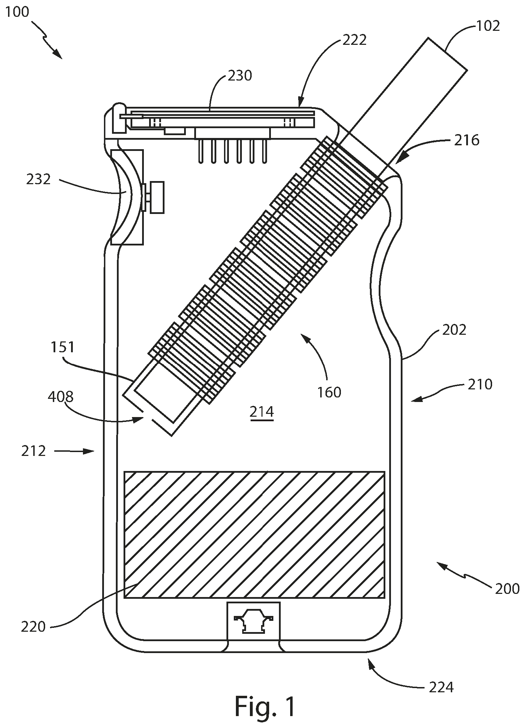

With reference to Figures 1-2E, the device 100 comprises a consumable-containing package 102 and an aerosol producing device 200. The device 100 generates aerosols through a heat-not-burn process in which a consumable-containing unit 104 is heated to a temperature that does not burn a consumable-containing unit 104 within the consumable-containing package 102, but does release the consumable from the consumable-containing unit 104 in the form of an aerosol product that can be inhaled. Thus, a consumable-containing unit 104 is any product that contains a consumable that can be released into aerosol form when heated to the proper temperature. Any description of the invention to a specific application, such as to a tobacco product, is provided only as a concrete example, and is not intended to be limiting. As such, the invention is not limited to use with tobacco products only.

**Consumable-Containing Package**

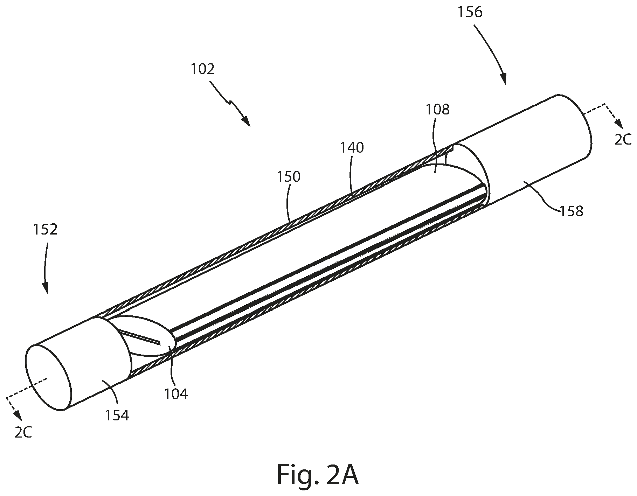

With reference to Figures 2A-6B, the consumable-containing package 102 is the component that is heated to release the consumable in aerosol form. The consumable-containing package 102 comprises a consumable-containing unit 104, and a metal (also referred to as the susceptor) 106 surrounding the consumable-containing unit 104 for heating the consumable-containing unit 104 from the inside out through an inductive heating system. In some embodiments, the consumable-containing package 102 can have an encasement 108 to contain the consumable-containing unit 104 and the susceptor 106. How well the consumable-containing package 102 is heated is dependent on product consistency. Product consistency takes into consideration various factors, such as the position, shape, orientation, composition, and other characteristics of the consumable-containing unit 104. Other characteristics of the consumable-containing unit 104 may include limiting the amount of oxygen contained in the unit. The goal is to maximize product consistency by keeping each of these factors consistent in the manufacturing process.

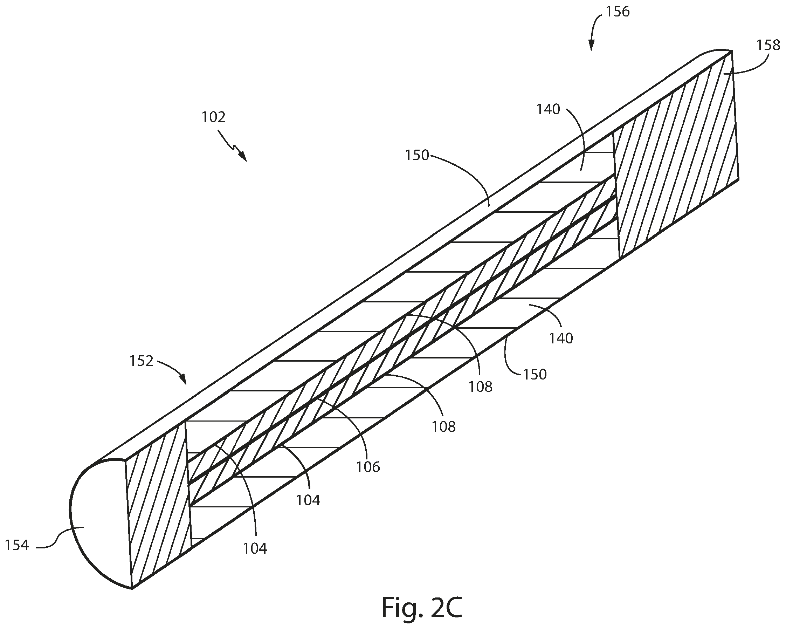

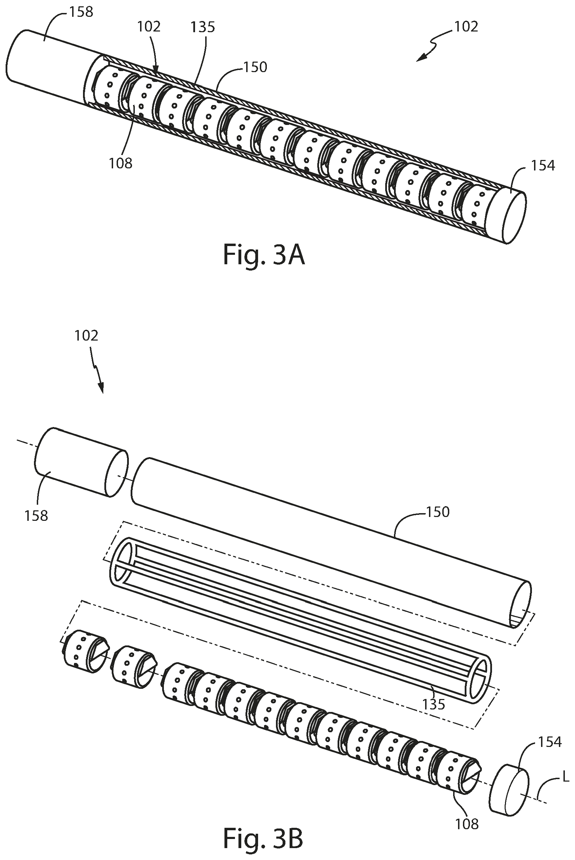

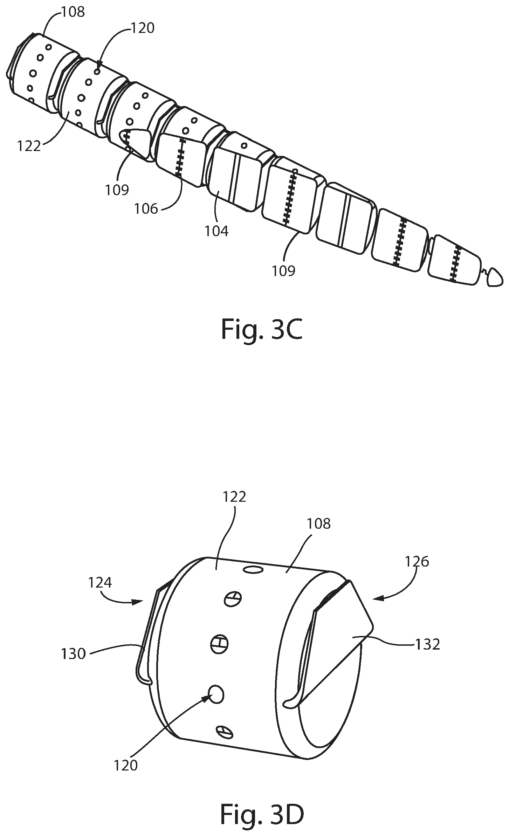

The encasement 108 is configured to be permeable to the aerosol to allow the aerosol to escape from the encasement 108. The consumable-containing unit 104 can be placed inside a housing 150. The housing 150 is less permeable or impermeable to the aerosol. The housing 150 can mimic a cigarette. As such, the housing 150 can be an elongated structure having a first end 152 and a second end 156 opposite the first end 152. When the consumable-containing unit 104 is heated by the susceptor 106, an aerosol is created containing the consumable. When the user draws on the housing 150, for example, by sucking on the second end 156, the aerosol escapes from the encasement 108, but not the housing 150. Due to the negative pressure created by the sucking at the second end 156, the aerosol is drawn towards the second end 156 through any space between the consumable-containing unit 104 and the housing 150. In some embodiments, a spacer 135 (see Figure 3A-3B) may surround the encasement 108, separating the housing 150 from the encasement 108. In such an embodiment, the aerosol can travel along the space created by the spacer 135. In some embodiments, the spacer 135 can be a filter 140 (see Figure 2C-2D). The aerosol producing device 200 is configured with the components for holding the housing 150 in the proper position to heat the susceptor 106.

If the form of the consumable-containing unit 104 is in direct physical contact with the susceptor 106 with maximal contact area between each, then it can be inferred that the thermal energy induced in the susceptor 106 will be largely transferred to the consumable-containing unit 104. As such, the shape and arrangement of the consumable-containing unit 104 relative to the susceptor 106 is an important factor. In some embodiments, the consumable-containing unit 104 is generally cylindrical in shape. As such, the consumable-containing unit 104 may have a circular or oval-shaped cross-section.

**The Consumable-Containing Unit**

The design of the consumable-containing unit 104 is to minimize the amount of air to which the consumable-containing unit 104 is exposed. This eliminates or mitigates the risk of oxidation or combustion during storage or during the heating process. As a result, at certain settings, it is possible to heat the consumable-containing unit 104 to temperatures that would otherwise cause combustion when used with prior art devices that allow more air exposure.

As such, in some embodiments, the consumable-containing unit 104 is made from a powdered form of the consumable that is compressed into a hard, compressed pellet or rod. Compression of the consumable reduces the oxygen trapped inside the consumable-containing unit 104, and limits migration of oxygen into the consumable-containing unit 104 during heating.

For example, the consumable-containing unit 104 may be one elongated unit defining a longitudinal axis L in the form of a rod or stick as shown in Figures 2A-2E. The consumable-containing unit 104 may be an elongated cylinder or tube having a circular transverse cross-section, an oval transverse cross-section, a rectangular transverse cross-section, and the like. In some embodiments, the consumable-containing unit 104 may be a plurality of cylindrical tablets or pellets stacked on top of one another as shown in Figures 3A-4B. As such, the consumable-containing unit 104 may be defined by two opposing ends 105, 107 and a sidewall 109 therebetween extending from the first end 105 to the second end 107 defining the length of the consumable-containing unit 104.

The susceptor 106 may be similarly elongated and embedded in the consumable-containing unit 104, preferably, along the longitudinal axis L and extending substantially the length and width (i.e. the diameter) of the consumable-containing unit 104. In consumable-containing units 104 having an oval cross-section, the diameter refers to the major diameter defining the long axis of the oval.

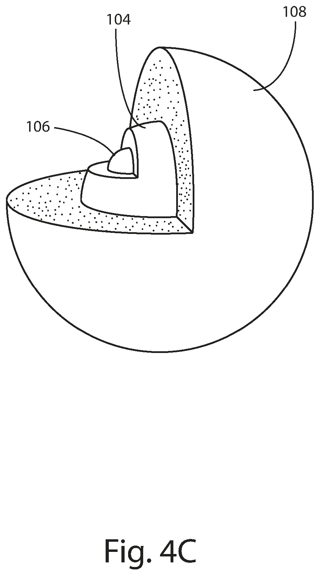

In some embodiments, as shown in Figure 4C, the consumable-containing unit 104 can take on any other shape, including spherical, ovoid, elliptical, and even amorphous. In general, the susceptor 106 can pattern the shape of the consumable-containing unit 104 to maximize the surface area contact between the susceptor 106 and the consumable-containing unit 104; however, the susceptor 106 can other shapes as well, including a plurality of susceptors 106 being sporadically distributed within the consumable-containing unit 104.

In an alternative embodiment, the consumable may be mixed with a substance that does not interfere with the function of the device 100, but displaces air in the interstitial spaces of the consumable and/or surrounds the consumable to isolate it from the air. For example, the consumable-containing unit 104 may further comprise an additive, such as a humectant, flavorant, filler to displace oxygen, or vapor-generating substance, and the like. The additive may further assist with the absorption and transfer of the thermal energy as well as eliminating the oxygen from the consumable-containing unit 104.

In yet another alternative embodiment, the consumable could be formed into tiny pellets, grains, powder, or other form that can be encapsulated to further reduce the air available to the consumable.

In some embodiments, the consumable-containing unit 104 can comprise a ground up source of the consumable made into powder form, then combined with a susceptor 106 by compressing tightly around the susceptor 106. By way of example only, the source of the consumable may be a plant, seed, flower, root, leaf, plant component, or any other source from which the consumable can be extracted. These components can be dried, ground up, and mixed with other components known for creating pellets and tablets to compress around a susceptor to form the pellet, tablet, or rod around the susceptor 106. The compressed pellet, tablet, or rod can be encased inside the encasement 108 to form the consumable-containing package 102.

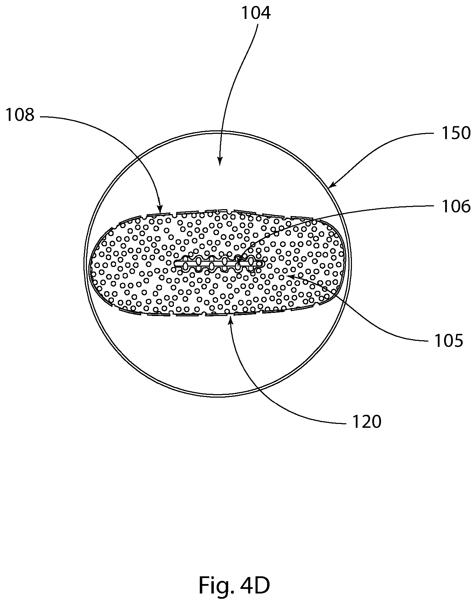

In some embodiments, the consumable can be extracted from its source and incorporated into a new medium 105 for carrying the consumable as shown in Figure 4D. This embodiment can be used, for example, if the consumable is not plant-based material, has been extracted from its natural source, was synthesized, or is not conducive for use as a compressed solid. The medium 105 containing the consumable forms the consumable-containing unit 104. For example, the medium 105 may be cotton, fiber glass, fabric, paper, pulp, fibrous material, and the like. The consumable can be combined with the medium 105 to form the consumable-containing unit 104, and then pressed tightly against the susceptor 106. While in the compressed form, the medium 105 can be bound inside the encasement 108 to maintain its compressed form to create the consumable-containing package 102. The consumable can be incorporated into the medium 105 via known carriers such as liquids, gels, resins, semi-solids, and the like. For example, a formulation for the consumable can comprise propylene glycol alginate, glycerin, alcohol, water, propylene glycol, propylene alginate, polysorbates and the like to bind the natural ingredient and some form of binding medium that may be composed of other non-reactive, natural, herbal materials or certain other biodegradable, non-reactive materials, e.g, paper, pulp, cotton, and the like. In some embodiments, the consumable can be incorporated into the medium 105 as a loose solid, for example, powder, grains, granules, and the like. In some embodiments, these loose solid consumables can be applied to the susceptor 106 and encapsulated in the encasement 108. In some embodiments, the medium 105 may further comprise an additive, such as a humectant, flavorant, filler to displace oxygen, or vapor-generating substance, and the like. The additive may further assist with the absorption and transfer of the thermal energy as well as eliminating the oxygen from the consumable-containing unit 104.

In some embodiments, the consumable may be formed into a resin, loose solid, pellets, pulp, paste and other appropriate forms for extrusion through an extruder. In these forms the extruder can be designed to be able to extrude on multiple sides of the susceptor 106, so as to center the susceptor 106 between layers while being extruded.

## The Susceptor

The susceptor 106 is the component that is heated through the inductive method and heats the consumable-containing unit 104 from the inside out. As such, the susceptor 106 is made of a metal that can be heated through an inductive method, such as ferrous metals.

The susceptor 106 can be machine extruded. Once extruded, the consumable-containing unit 104 can be combined with the susceptor 106 by compressing it around the susceptor 106 along the length of the susceptor 106. Alternatively, the susceptor 106 could be stamped from flat metal stock or any other suitable method of fabrication prior to assembling the consumable containing unit 104 around the susceptor 106.

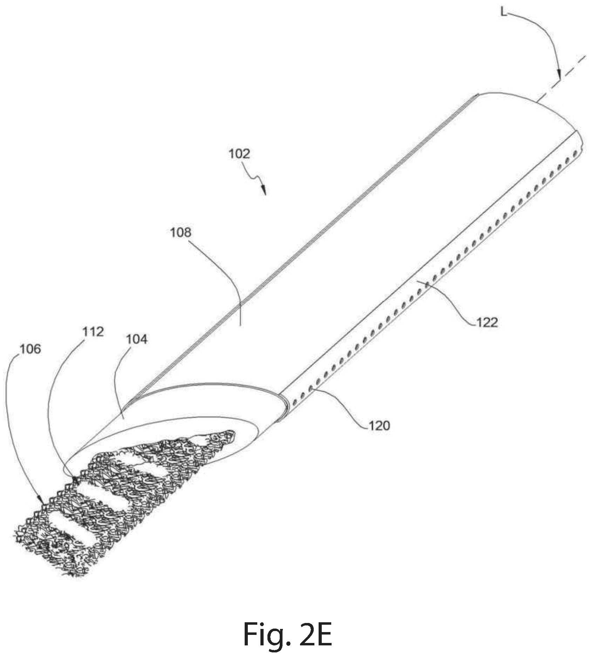

In some embodiments, as shown in Figure 2E, the susceptor 106 may be made of steel wool. For example, the susceptor 106 may be comprised of fine filaments of steel wool bundled together in the form of a pad. As such, the steel wool pad comprises numerous fine edges. In some embodiments, the steel wool pad may be doused with, immersed in, or fully filled with an additive, such as a humectant, flavorant, vapor-generating substance, a substance to retard oxidation of the steel wool (rust), and/or a filler to eliminate air between the steel wool filaments, and the like. As shown in Figure 2E, there may be cut-outs or gaps 112 along the steel wool pad to divide the consumable containing unit 104 into discrete segments for individual heating, as described below. Alternatively, individual pads of steel wool may be used, separated by space and/or consumable, so that each pad may be heated individually during use.

The steel wool can be made from low-carbon steel. Advantages of the steel wool, include, but are not limited to, easy disposability from an environmental standpoint in that it begins to oxidize soon after it is heated; and thereby, becomes friable and degrades easily without dangerous sharp edges. Also, metals composed of iron and carbon are relatively non-toxic. Also, the carbon increases the stiffness.

Preferably, the susceptor 106 is flattened until it is paper-thin. As such, the thickness T of the flattened susceptor 106, particularly the steel wool, can be less than 0.1 inch. (2.54 mm) Preferably, the thickness of the susceptor 106 can be less than 0.05 inch. (1.27 mm) More preferably, the thickness of the susceptor 106 can be less than 0.025 inch, (0.635 mm) or even less than 0.01 inch. (0.254 mm) In some embodiment, the susceptor 106 can be as thin as 0.0039 inch. (0.099 mm). The susceptor 106 can range in length from about 0.5 inch (12.7 mm) to about 1.25 inches. (31.75 mm) Preferably, the susceptor is about 0.75 inch (19.05 mm) in length.

To achieve a thin, flattened strip of steel wool, a piece of steel wool may undergo a series of stretching and compressing until the desired thickness T is achieved. This would be accomplished via a rolling compression followed by a stamping process to get the requisite shape and texture.

Once the desired thickness is achieved, the susceptor 106 can be cut into the desired shape and dimensions. Use of the steel wool, and making the steel wool thin, allows for easy and prolonged cutting as the blade for cutting the steel wool can last longer compared to traditional metals and thicker susceptors currently on the market. Furthermore, using steel wool is cheaper to make and requires less energy to heat up. In some embodiments, approximately one-third less energy is required to reach the same temperature of other non-steel wool susceptors.

In some embodiments, a consumable-containing unit 104 can be combined with or incorporated into a susceptor 106 by co-extruding it with the susceptor 106 to create a layer of the consumable-containing unit 104 on top or on bottom of the layer of the susceptor 106. In some embodiments, two layers of the consumable-containing unit 104a, 104b can be co-extruded with the susceptor 106 in between to create a sandwich around the susceptor 106 that can be compressed in between the two layers of the consumable-containing unit 104a, 104b.

In some embodiments, in which the consumable-containing unit 104 comprises a medium 105 containing the consumable, the susceptor 106 can be packaged or surrounded by the medium 105, and then the medium 105 and the susceptor 106 can be compressed together and placed inside an encasement 108 (see Figure 4D).

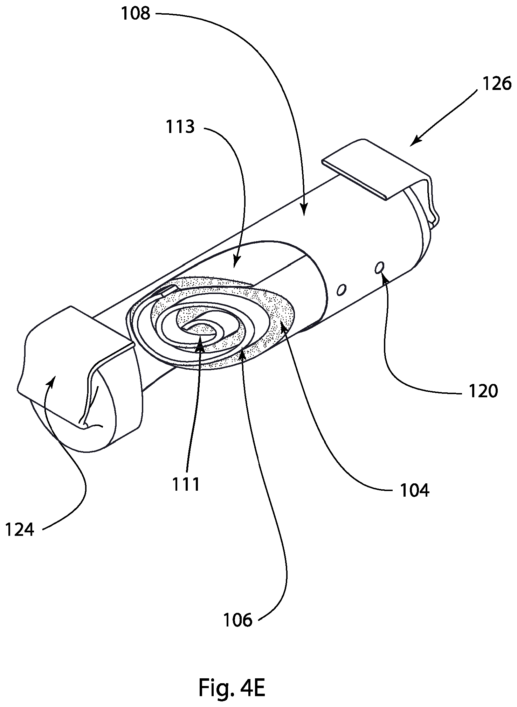

In some embodiments, the consumable-containing unit 104 and the susceptor 106 having similar dimensions can be placed one on top of the other. For example, this may occur through a co-extrusion process. The consumable-containing unit 104 and the susceptor 106 can then be rolled from a first end 111 to a second end 113 the way a sleeping bag is rolled up as shown in Figure 4E. Preferably, the susceptor 106 is on top during the rolling process. The result is that the susceptor 106 and consumable-containing unit forms a cylindrical shape defining a longitudinal axis L, with a spiraling pattern when view along a transverse cross-section, but the susceptor 106 remains internal to the consumable-containing unit 104. The transverse cross-section is a cut along the diameter of the cylinder perpendicular to the longitudinal axis. This configuration further increases surface area exposure between the susceptor 106 and the consumable-containing unit 104. The susceptor 106 can have a plurality of holes 110 to allow the aerosol to escape.

In some embodiments, in which the susceptor 106 is steel wool or other metal having the porous characteristics of steel wool, the consumable can be incorporated directly into the susceptor 106, for example, in a fluid (e.g., liquid, semi-liquid, viscous substance, and the like) or loose solid (e.g., powder, grains, granules, and the like) form, in which case the susceptor 106 has the dual function of being the heating element and the consumable-containing unit 104. As such, the consumable-containing unit 104 can be the susceptor 106 combined with the consumable incorporated therein.

The susceptor 106 can be made of any metal material that generates heat when exposed to varying magnetic fields as in the case of induction heating. Preferably, the metal comprises a ferrous metal. To maximize efficient heating of the consumable-containing unit 104, the susceptor 106 generally matches the shape of the largest cross-sectional area of the consumable-containing unit 104 so as to maximize the surface area with which the consumable-containing unit 104 comes into contact with the susceptor 106, but other configurations may also be used. In the embodiments in which the consumable-containing unit 104 is an elongated cylinder, the largest cross-sectional area would be defined by dividing the elongated cylinder down the longitudinal axis L along its major diameter creating a rectangular cross-sectional area. As such, the susceptor 106 would also be rectangular with dimensions substantially similar to the dimensions of the cross-sectional area of the elongated cylinder.

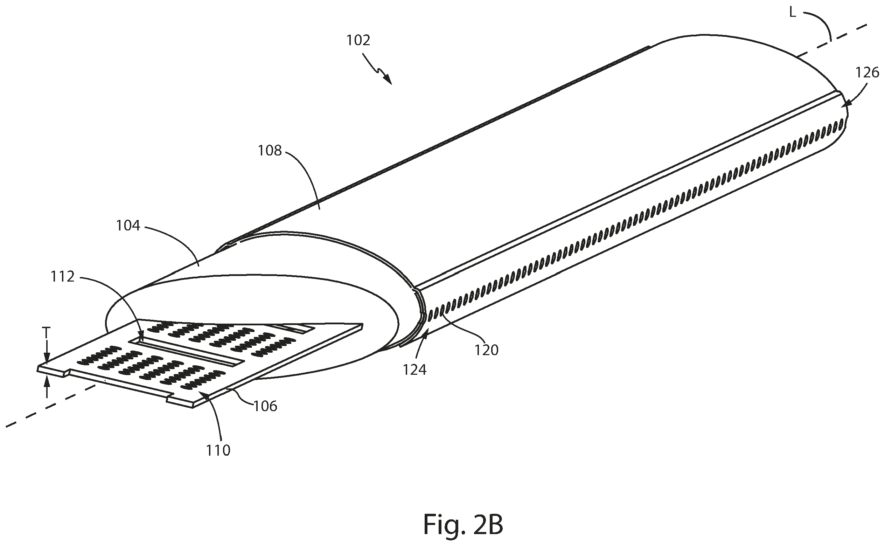

In some embodiments, the susceptor 106 may be a metal plate. In some embodiments, the susceptor 106 may be a metal plate with a plurality of openings 110 as shown in Figure 2B, like a mesh screen. Inductive heating appears to be most effective and efficient at the edges of the susceptor 106. A mesh screen creates more edges in the susceptor 106 that can contact the consumable-containing unit 104 because the edges define the openings 110.

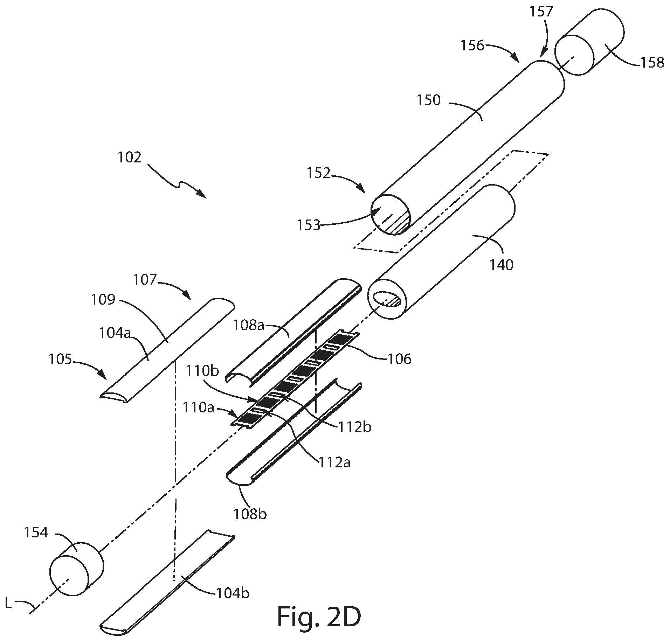

As shown in Figure 2D, the susceptor 106 may be a strip patterned with an array of small openings 110 to increase the amount of edges that can be utilized in an efficient inductive heating process, followed by a larger gap 112 that allows for that length of the susceptor 106 that will not allow for inductive heating, or at least mitigate inductive heating and/or mitigate conduction from the segment being heated. This configuration allows for the consumable-containing package 102 to be heated in discrete segments. The elongated susceptor 106 may be an elongated metal plate having a longitudinal direction, the elongated metal plate comprising sets of openings 110a, 110b and sets of gaps 112a, 112b wherein the sets of openings 110a, 110b alternate in series with the sets of gaps 112a, 112b along the longitudinal direction of the elongated metal plate such that each set of openings 110a, 110b is adjacent to one of the gaps 112a, 112b. Therefore, moving from one end of the susceptor 106 to the opposite end, there is a first set of openings 110a, then a first gap 112a, then a second set of openings 110b, then a second gap 112b, and so on. In the area of the gaps 112, there is very little metal material; therefore, there is minimal heat transfer. As such, even though the consumable-containing unit 104 is a single unit, it can still be heated in discrete sections. The consumable-containing unit 104 and susceptor 106 can then be wrapped in with the susceptor 106 can be placed into an encasement 108.

## The Encasement

In some embodiments, the encasement 108 may be made of metal. As such, the encasement can be molded, or hand-crafted and tooled. The preferred metal can be aluminum with pre-punched openings 120. In some embodiments, the aluminum may be lined with porous paper, or nonporous paper with vent holes to allow the aerosol to escape from the encasement 108.

The consumable-containing unit 104 is placed inside the encasement 108 to contain the heat generated by the susceptor 106. Openings 120 in the encasement 108 can allow the consumable aerosol to escape when heated. Because the openings 120 create an avenue through which air can enter into the encasement 108 to be exposed to the consumable-containing unit 104, the openings 120 may be temporarily sealed using a coating. The coating is preferably made of a composition that melts at temperatures that create consumable aerosols. Therefore, as the susceptor 106 is heated, due to the lack of air inside the encasement 108, the consumable-containing unit 104 can be raised to exceedingly high temperatures without combusting. As the susceptor 106 reaches high temperatures, the consumable aerosols that begin to form, are not able to escape. When the coating melts away and exposes the opening 120, then the consumable aerosols are able to escape the encasement 108 for inhalation. In the preferred embodiment, the coating may be propylene glycol alginate ("PGA") gel. The coating may also include a flavoring. Therefore, as the coating melts away and the consumable aerosol is released, the flavoring is also released with the consumable aerosol. In some embodiments, the flavoring can be mixed with additives.

In some embodiments, the openings 120 may be a plurality of holes or slits. The openings 120 may be formed along the length of the sidewall 122 of the encasement 108, arranged radially around the sidewall 122, arranged randomly or uniformly throughout the sidewall 122, and the like. In some embodiments, the openings 120 may be a plurality of holes along the opposite ends 124, 126 of the encasement 108. In some embodiments with the elongated consumable-containing unit 104, the encasement 108 may also be elongated with the opening 120 in the form of one or more elongated slits traversing the length of the encasement parallel to the longitudinal axis L, thereby creating a seam. That seam may be folded or crimped, but still leave a gap through which consumable aerosols may travel, either along its entire length or in discrete areas. Like the openings 120 described above, the seam may be sealed with a coating.

In some embodiments, the encasements 108 may be made of a two piece unit having a first encasement section 108a and a second encasement section 108b. The consumable-containing unit 104 can be inserted into the first encasement section 108a and the second encasement section 108b may be placed on top of the first encasement section 108a to cover the consumable-containing unit 104. Preset openings 120 can be formed into the encasement 108 prior to encapsulating the consumable-containing unit 104.

Having established the general principles of the consumable-containing package 102, variations have also been contemplated that achieve the same objectives. For example, in some embodiments, the consumable-containing unit 104 may comprise two elongated sections 104a, 104b. The two elongated sections 104a, 104b of the consumable-containing unit 104 may be defined by a plane parallel to and cutting through the longitudinal axis L along the diameter. Therefore, the two elongated sections 104a, 104b may be half-cylinder sections that when mated together form a full cylindrical consumable-containing unit 104.

In some embodiments, as shown in Figures 3A-4B, the consumable-containing unit 104 may be in the form of pellet or tablet. Unlike the consumable-containing unit 104 that is an elongated cylinder or tube in which the length of the sidewall 109 is much longer than the diameter, in the tablet embodiment, the tablet may be a short cylinder defining a longitudinal axis L, wherein the length of the sidewall 109 is closer to the size of the diameter, or shorter than the diameter. The susceptor 106 may have a flat, circular shape to match the cross-sectional shape of the tablet when cut transversely, perpendicular to the longitudinal axis L. The consumable-containing unit 104 can be compressed about the susceptor 106. To mimic a cigarette, a plurality of the consumable-containing units 104 can be stacked, end-to-end along their longitudinal axes L, to form an elongated cylinder. Therefore, each individual consumable-containing unit 104 can be heated separately, effectively mimicking the segments of the consumable-containing unit 104 having an elongated, tubular body.

Other shapes can also be used, such as square or rectangular with a susceptor 106 having a corresponding shape. The cylindrical shape, however, can be easy to conform into a shape that mimics the shape of an actual cigarette.

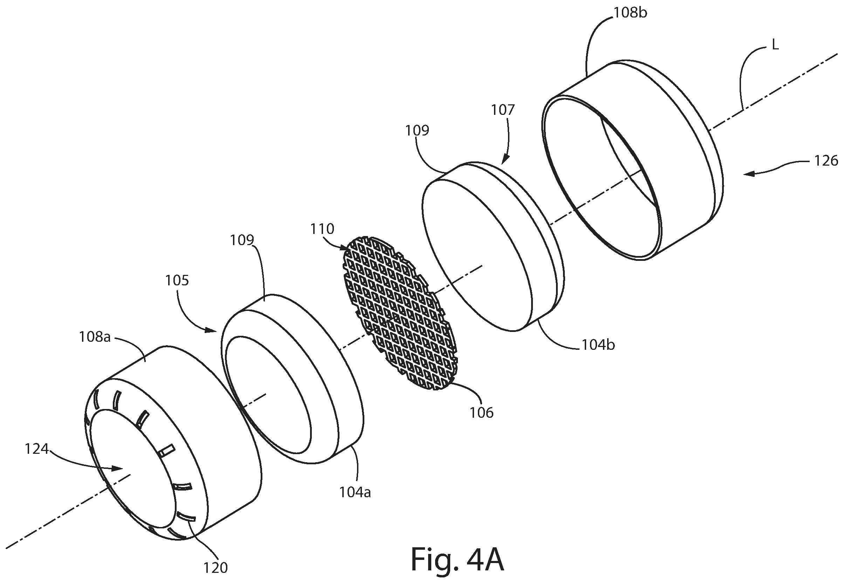

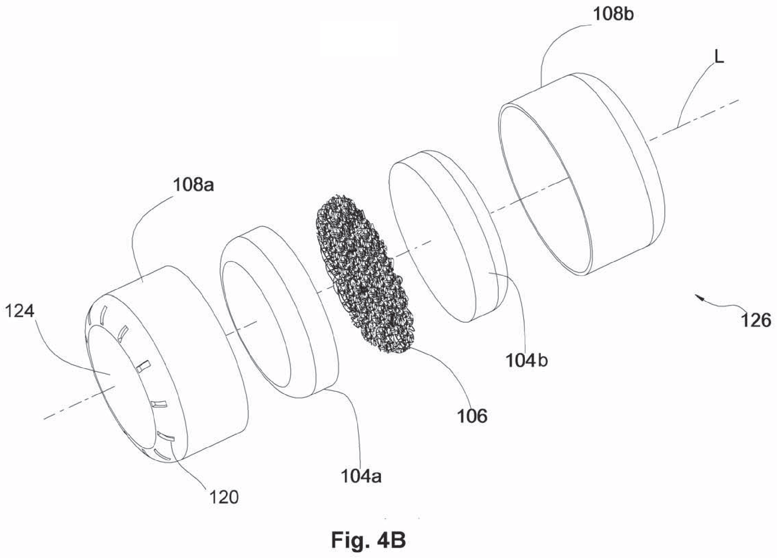

In some embodiments, the consumable-containing unit 104 may be formed from two sections 104a, 104b of the consumable-containing unit 104 combined together to make a whole, as shown in Figures 4A and 4B. The two sections 104a, 104b are defined by splitting the consumable-containing unit 104 in half transversely along a plane perpendicular to the longitudinal axis L. The susceptor 106 may be sandwiched in between the two sections 104a, 104b. With the susceptor 106 sandwiched in between the two consumable-containing sections 104a, 104b, the consumable-containing unit 104 can be enclosed by the encasement 108. This process can be repeated to create a plurality of individual consumable-containing units 104 sandwiching respective susceptors 106, each individually contained in a respective encasement 108. The plurality of consumable-containing units 104 may be stacked, one on top of the other to create the consumable-containing package 102 in which each individual consumable-containing unit 104 may be heated individually, one at a time.

In some embodiments, the encasement 108 may be aluminum wrapped around a consumable-containing unit 104. The aluminum can have excess folds 130, 132 at opposite ends as shown in Figure 3D. These excess folds 130, 132 create a gap in between adjacent consumable-containing units 104 when stacked on top of each other.

In some embodiments, the encasement 108 may be two-pieces having a first encasement section 108a and a second encasement section 108b that serves as a covering or cap to enclose the consumable-containing unit 104 inside the first encasement section 108a, as shown in Figures 4A and 4B. As described previously, the openings 120 on the encasement 108 may be along the sidewall 122 or at the ends 124, 126. As described previously, the susceptor 106 may be any type of metal that is subject to induced heating, including steel wool as shown in Figure 4B. In the preferred embodiments, numerous edges are created in the susceptor 106 by creating a plurality of holes 110 or using steel wool filaments compressed together. The steel wool filaments may be fine to medium grade. As discussed above, the steel wool pad may be soaked in, coated, or filled with additive, flavorant, protectant, and/or filler.

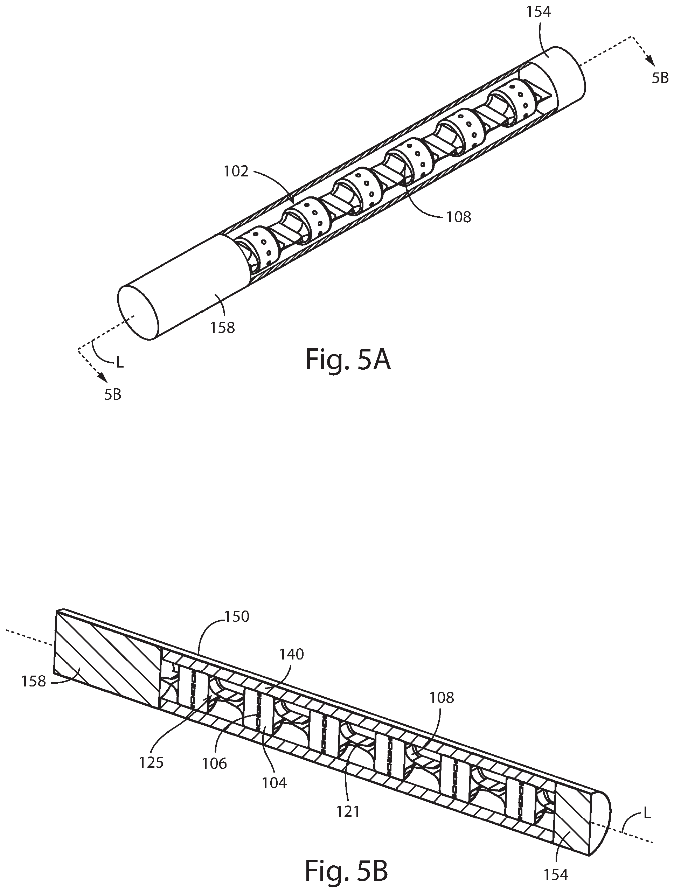

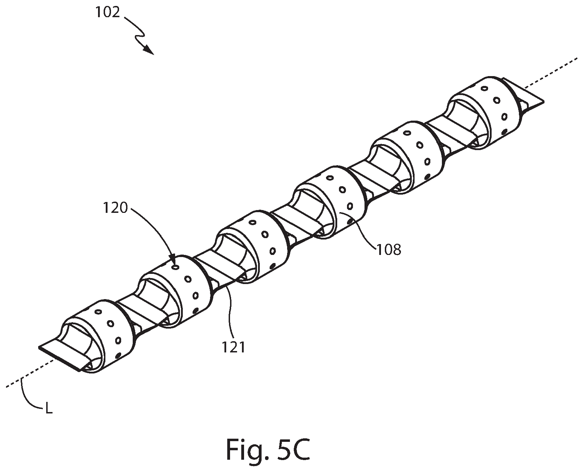

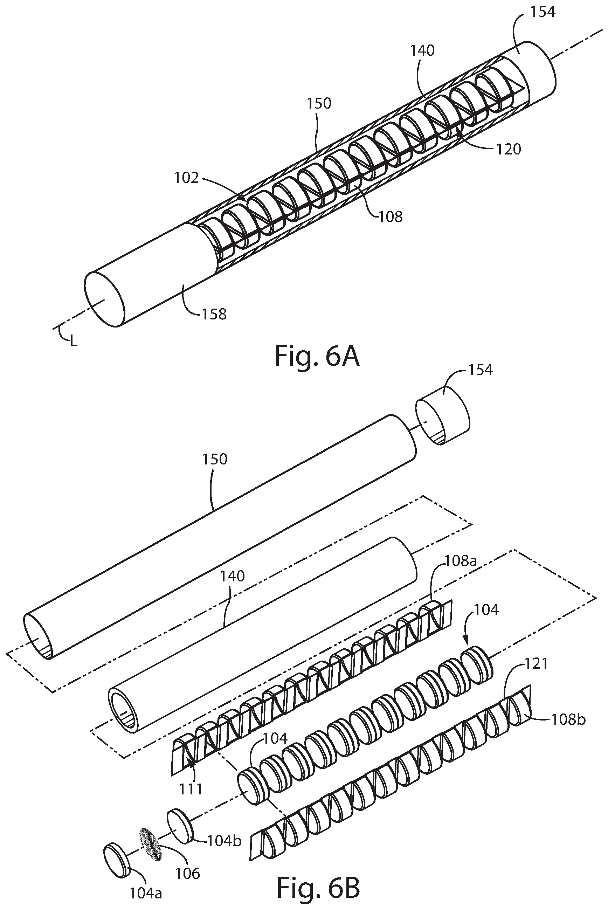

In some embodiments, a plurality of consumable-containing units 104 may be contained in a single elongated encasement 108, as shown in Figures 5A-6B. The encasement 108 may be molded with compartments 111 to receive each individual consumable-containing unit 104. In some embodiments, the individual compartments 111 may be connected to each other by a bridge 121. In some embodiments, the bridge 121 may define a channel 125 that allows fluid communication from one compartment 111 to another. In some embodiments, the bridge 121 may be crimped to prevent fluid communication between one compartment 111 and the other through the bridge 121. In some embodiments, the elongated encasement 108 may be a two-piece assembly split transversely along the longitudinal axis L, as shown in Figures 6A-6B. The consumable-containing units 104 can be seated in the compartments 111 of one of the encasement sections 108a. The second encasement section 108b can then be mated to the first encasement section 108a to cover the consumable-containing units 104. The split between the first encasement section 108a and the second encasement section 108b can be used as the opening 120. Alternatively, preset openings 120 can be formed in one or both of the encasement sections 108a, 108b.

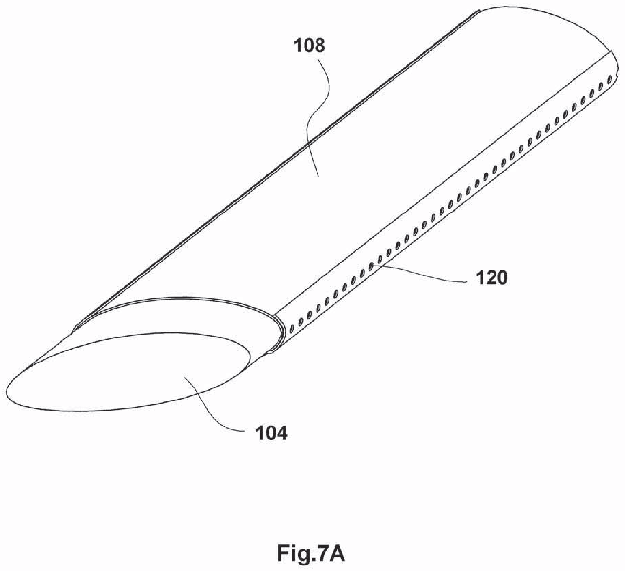

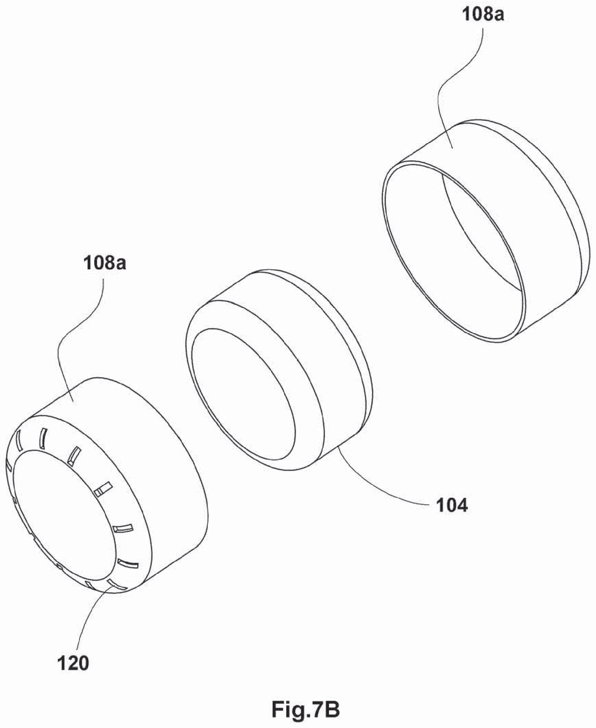

In some embodiments, as shown in Figure 7A-7D, the encasement 108 may be made out of material that allows the encasement 108 to serve as the susceptor. For example, the encasement 108 can be made of steel, or otherwise comprise ferrous metal, or any other metal that can be heated using induction heating. In such an embodiment, an interior susceptor 106 would not be required to be embedded into the consumable-containing unit 104. The encasement 108 can still comprise a plurality of holes 120, and be covered with an additive and/or sealant such as PGA. Such an embodiment can be made into an elongated tube as shown in Figure 7A or into tablets or disks as shown in Figure 7B. The encasement 108 can be a two piece encasement having a first encasement section 108a and a second encasement section 108b as discussed previously.

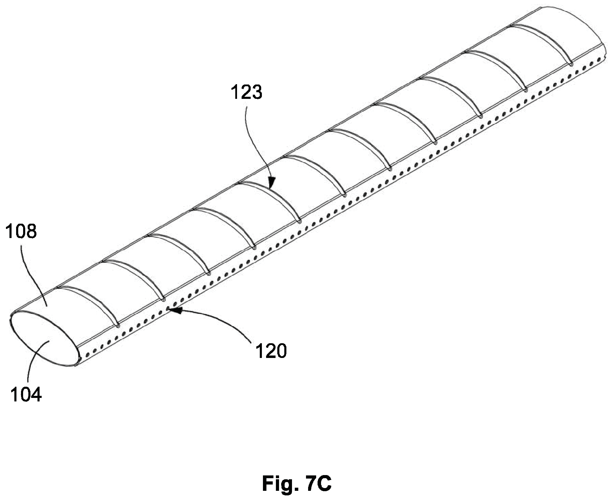

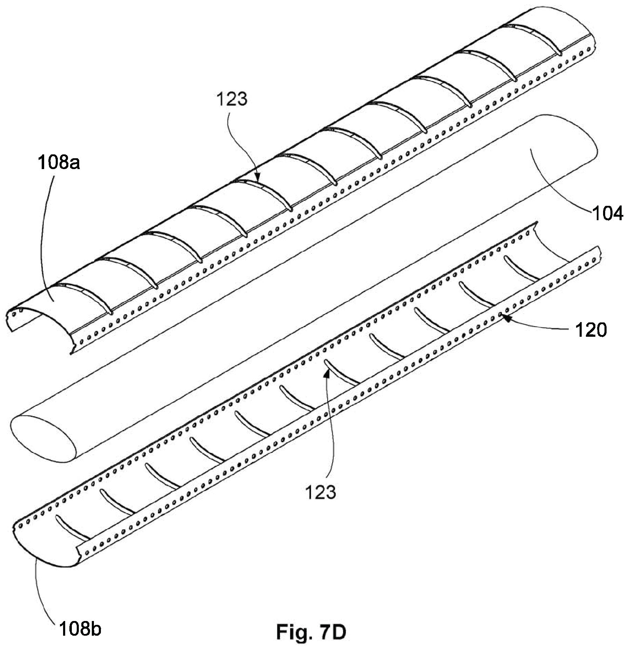

In some embodiments, the encasement 108 may have transverse slits 123 transversely across the encasement 108, generally perpendicular to the longitudinal axis L as shown in Figures 7C and 7D. The slits 123 create segmentation in the encasement 108 so that only a small segment of the consumable-containing unit 104 is heated per actuation. The transverse slits 123 may be through holes, which expose the consumable-containing unit 104 underneath. In such embodiments, the segments may be filled with a coating or some other plug to seal the hole, either permanently or with a substance that will melt upon heating and allow the aerosol to escape through the slit 123. In some embodiments, the plug may be made from material that can function as a heat sink and/or a substance that is not easily heated via induction to reduce the heating effect at the transverse slits 123. In some embodiments, the transverse slit 123 may be a recessed portion of or an indentation in the encasement 108. In other words, the transverse slit 123 may be a thinned portion of the encasement 108. As such, the transverse slit 123 may define a well. The well can be filled with a plug that can function as a heat sink and/or a substance that is not easily heated via induction to reduce the heat transfer along the transverse slit 123.

In some embodiments, the encasement 108 can be a hard shell to provide structural integrity to the consumable-containing package 102. In some embodiments, the encasement 108 can be a pliable wrapper and the consumable containing unit 104 and susceptor 106 can be wrapped inside the encasement 108. For example, in some embodiments, the susceptor 106 can be a flat sheet of wool, wrapped around with cotton in which the consumable has been incorporated in liquid or loose solid form. The wool and cotton can be wrapped with an aluminum foil containing a plurality of holes 120.

In some embodiments, the encasement 108 may be made of porous material as shown in Figure 4C. For example, the encasement 108 may be a porous paper wrap, or other similar material. As such, the pores would function as the openings 120 by allowing the aerosol from the consumable-containing unit 104 to escape when heated. Furthermore, because the consumable-containing unit 104 is compressed so as to eliminate the oxygen, combustion is still unlikely at the working temperatures and the short duration of time the consumable-containing unit 104 is exposed to the high heat. Even though the exterior of the encasement 108 is exposed to oxygen along the channel between the encasement 108 and the housing 150, the inductive heating rapidly heats the consumable from the inside-out, so the exterior of the encasement 108 never reaches combustion temperature. For example, at temperatures greater than 350 degrees Celsius and even higher at 400 degrees Celsius, combustion did not occur in the present invention. To the contrary, because other devices use loose tobacco (or consumable material), or heat the consumable from the outside in, these devices are susceptible to burning the consumable. Additionally, other heated tobacco products heat all the tobacco at one time-either from the inside out with a blade/rod or via an "oven" surrounding the consumable. This causes a tired burnt taste after as few as 3 puffs. In addition, most other current heated tobacco technologies route the airflow through the tobacco, which lowers the combustion temperature. In contrast, in the present invention the heat source is the susceptor 106, which is encased by the compressed consumable-containing unit 104 to result in an oxygen-free or oxygen-restricted environment, and the consumable aerosol produced by the heating is expelled from that environment and into the surrounding channel between the between the encasement 108 and the housing 150.

In embodiments in which the encasement 108 is a porous material, the encasement 108 can be thin allowing the aerosol to pass through the pores of the encasement and exit the encasement 108 laterally or radially outwardly. This allows the aerosol to enter the channel created between the housing 150 and the encasement 108. In some embodiments, the encasement 108 may be thicker and have sufficient porosity to allow the aerosol to travel through the pores longitudinally along the length of the encasement 108. In such an embodiment, a channel may not be needed between the housing 150 and the encasement 108. Such porous materials may include cigarette paper, cellulose or other filter media, or any suitable material for the purpose.

In some embodiments, the encasement 108 can comprise a coating 115. As used herein, a coating 115 is a fluid, such as a liquid, semi-liquid, or viscous substance, that has hardened into a shell, and in particular, a rigid, porous shell that can maintain its shape when handled. An example of a coating 115 is a batter made from starch. The starch can be corn starch, potato starch, or starch from any other plant source, such as sago, wheat, barley, rice, tapioca, cassava, and the like, and in some embodiments may be combined with other fiber-like materials such as paper pulp or the like. When starch is mixed with a fluid, such as water, the batter becomes a liquid, semi-liquid batter, or viscous. With heat and/or time, the batter can harden. This is a common technique used in cooking fried foods. Similarly, the coating 115 in fluid form can be applied to the consumable-containing unit 104. For example, in fluid form, the batter can be sprayed, or painted on to the consumable-containing unit 104, or the consumable-containing unit 104 can be dipped into the batter. Heat can be applied to the coated consumable-containing unit 104. The coating then creates a hardened, yet porous shell around the consumable-containing unit 104 to form the encasement 108. In another embodiment, a shell-like encasement can be molded using the starch pulp combination. The oxygen, however, is still largely eliminated between the consumable and the susceptor 106.

In such an embodiment, holes 120 need not be punched into the encasement 108 due to the porosity of the encasement 108. Porosity may be created and/or increase as the encasement 108 is heated during use. In addition, starch allows for a high flash point, and does not add flavor. Furthermore, with starch, there is very little risk of leakage, and starch can have a long shelf life.

The encasement 108 containing the consumable-containing unit 104 can then be inserted into the housing 150 to form the consumable-containing package 102. When the susceptor 106 is heated, the consumable aerosolizes and escapes into the porous encasement 108. When the user draws on the mouthpiece 158, the negative pressure created inside the housing 150 causes and airflow in the direction of the mouthpiece 158, and the aerosolized consumable travels through the pores of the encasement towards the mouthpiece where the consumable can be inhaled by the user.

Like the embodiment shown in Figures 2A-2E, the encasement 108 in the form of a porous shell can be one elongated unit, such as tubes or rods. In some embodiments, as shown in Figures 3A-D, the porous shells can be discrete, short cylinder units or block units that are stackable. In some embodiments, as shown in Figure 4C, the porous shells can be spherical.

By way of example only, a starch powder can be sprayed on the active ingredient formulation around the susceptor, forming the porous encasement; or a batter can be formed by adding water to the starch powder to give it the consistency of honey and then the active ingredient and susceptor would be dipped in the batter and that would be allowed to dry, forming a porous encasement.

Therefore, a method of manufacturing a consumable-containing package 102 for use in an aerosol producing device 200 comprises combining a susceptor 106 with a consumable to form a consumable-containing unit 104; applying a coating onto the consumable-containing unit 104; heating the coating to create an encasement 108 around the consumable-containing unit 104, wherein the encasement 108 is porous, whereby the consumable-containing package 102 is produced. Preferably, the coating comprises starch. The method can further comprise extruding the consumable with the susceptor 106 to form the consumable-containing unit 104. The method can further comprise rolling the extruded susceptor 106 and consumable to form a cylinder with a spiraling pattern when viewed along a transverse cross-section. The method can further comprise incorporating the consumable into a medium 105 to form the consumable-containing unit 102.

In some embodiments, a method of manufacturing a consumable-containing package 102 for use in an aerosol producing device 200 comprises flattening a piece of steel wool into a susceptor 106 having a thickness of less than 0.1 inch (0.254 mm); combining the susceptor 106 with a consumable to form a consumable-containing unit 104; placing the consumable-containing unit 104 into an encasement 108, whereby the consumable-containing package 102 is produced. The method can further comprise extruding the susceptor 106 to flatten the piece of steel wool. The method can further comprise extruding the susceptor 106 with the consumable to combine the susceptor 106 with the consumable. The method can further comprise rolling the extruded susceptor 106 and consumable to form a cylinder with a spiraling pattern when viewed along a transverse cross-section. The method can further comprise incorporating the consumable into a medium 105 to form the consumable-containing unit 104.

In some embodiments, the encasement 108 may be eliminated altogether and the consumable-containing unit 104 is simply surrounded by the housing 150. In such an embodiment, the aerosol is released from the consumable-containing unit 104 directly into the channel between the consumable-containing unit 104 and the housing 150. Like the encasement embodiments, the exterior of the consumable-containing unit 104 does not reach combustion temperature due to the rapid inside-out inductive heating.

In embodiments where the encasement 108 is porous or absent, freshness of the consumable may be maintained by use of an airtight packaging, which may be filled with nitrogen or other inert gas to prevent oxidation. Such packaging could be used for large packages of consumable-containing packages 102, for example, with tobacco products where multiple consumable-containing packages 102 may be used each day. Alternatively, individual packaging may be used for consumable-containing packages 102 containing medicants, where the consumable is only used periodically.

**The Spacer**

The consumable-containing package 102 may comprise a spacer 135 to create a space between the housing 150 and the consumable-containing unit 104. The space between the housing 150 and the consumable-containing unit 104 creates a passageway for airflow to carry the consumable in aerosolized form to the mouthpiece 158 for inhalation.

In some embodiments, the spacer 135 can be a filter tube 140 to encapsulate the consumable-containing unit 104, susceptor 106, and the encasement 108 as shown in Figure 2C and 2D. In other words, the porosity of the filter tube 140 creates the passageway for airflow to the mouthpiece. By way of example only, the filter tube 140 may be made of cellulose or cellulose acetate, although any suitable filter material may be used.

The filter tube 140 may be made of filter material to capture any unwanted debris while allowing the consumable aerosol that is released from the heating of the encasement to pass transversely through the filter. The filter tube 140 may surround the encasement 108 and further cover the coated openings 120. Because the filter tube 140 may be made of filtering material, the consumable aerosol is able to travel through the filter tube 140. By way of example only, the filter tube may be made of cellulose or cellulose acetate, although any suitable filter material may be used.

In some embodiments, the spacer 135 can be any rigid structure that can maintain a space between the encasement 108 and the housing 150, while creating an airflow passageway from the encasement 108 to the mouthpiece 158 as shown in Figure 3A and 3B. For example, the spacer 135 can be a framework, corrugated material, rods, rings mounted on the encasement 108 with transverse holes cut through the wall of the ring, and the like.

In some embodiments, the spacer may be optional. For example, in embodiments in which the encasement is made from a rigid, but porous material, a spacer 135 would not be required because the porosity of the encasement 108 creates the airflow passageway for the consumable to travel from the consumable-containing unit 104 to the mouthpiece 158.

In some embodiments, the spacer 135 may not be necessary. Where the consumable-containing unit 104 and/or encasement 108 have a different shape than the housing 150, a natural channel is formed. For example, where the consumable-containing unit 104 and/or encasement 108 have a triangular or square cross-section, placing them into a cylindrical housing 108 creates three and four channels, respectively. For a cylindrical housing 150, any consumable-containing unit 104 and/or encasement 108 having a polygonal cross-section, or even an oval cross-section, will create channels for the aerosol without need for a spacer 135. Any cross-section that creates one or more channels may eliminate the need for a spacer 135. Alternatively, the consumable-containing unit 104 and/or encasement 108 could be cylindrical and the housing 150 could be polygonal, or even a square, to create channels without need for a spacer 135.

**The Housing**

The consumable-containing package 102 may further comprise a housing 150 to enclose the encasement 108, and the spacer 135, if any. In the preferred embodiment, the housing 150 generally mimics a cigarette. As such, the housing 150 is generally tubular having a first end 152 with a first opening 153, and a second end 156 opposite the first end 152, the second end 156 having a second opening 157. As such, the housing 150 can be a hollow tube configured to allow a user to draw on the second end 156 causing air to flow through the first opening 153 at the first end 152 downstream towards the second opening 157 at the second end 156. For example, the housing 150 may be about 1.5 inch (38.10 mm) to about 3.25 inches (82.55 mm) long.

The housing 150 comprises material that is less likely to allow the consumable aerosols to pass through, such as paper, plastic, metal, ceramic, glass, wood, carbon fiber, compressed starch paper, and the like. This allows the consumable aerosol to follow the path of inhalation towards the user's mouth in the direction of the first end 152 of the housing 150 to the second end 156 of the housing 150.

The housing 150 may be capped with an end cap 154 at the first end 152. The end cap 154 may be comprised of a type of filter material. As the air flows from the first end 152 towards the second end 156, the end cap 154 functions as a prefilter. Another function of the end cap 154 in the housing 150 is to serve as an airflow restrictor, producing negative pressure inside of the consumable when air is being drawn through the end cap 154.

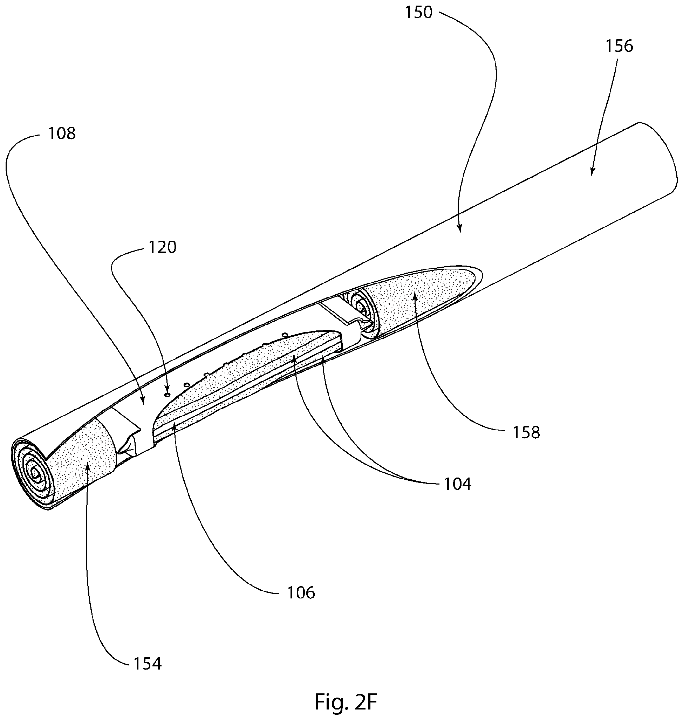

At the opposite end 156 of the housing 150 is a mouthpiece 158 that the user sucks on to draw the heated consumable aerosol out of the encasement 108 along the housing 150 towards the mouthpiece 158 and into the user's mouth. As such, the mouthpiece 158 may also comprise a type of filter, similar to that of the end cap 154. Any type of filter can be used as the end cap 154 and mouthpiece 158. In some embodiments, the end cap 154 and/or the mouthpiece 158 can be a sheet of filter material, paper, or any other suitable material, that has been rolled from a first end to a second end to create an internal spiral when viewed in transverse cross section as shown in Figure 2F.

The encasement 108 can be slid into the housing 150 through the first opening 153 or the second opening 157. If the encasement 108 is slid in from the first opening 153, the mouthpiece 158 can be put in place to prevent the encasement 108 form sliding out. Similarly, if the encasement 108 is slid into the housing 150 from the second opening 157, the end cap 154 can be put in place to prevent the encasement 108 from sliding out the first opening 153. As such, the mouthpiece 158 and the end cap 154 keep the encasement 108 in place inside the housing 150.

The housing 150 wrapped around the filter tube 140 creates a longitudinal channel through the filter tube 140 through which the consumable aerosol travels, rather than escaping radially out the filter tube 140. This allows the consumable aerosol to follow the path of inhalation towards the user's mouth. One end 152 of the housing 150 may be capped with an end cap 154. The end cap 154 may be comprised of a type of filter material. At the opposite end 156 of the housing 150 is a mouthpiece 158 that the user sucks on to draw the heated consumable aerosol out of the encasement 108 along the filter tube 140 towards the mouthpiece 158 and into the user's mouth. As such, the mouthpiece 158 may also be a type of filter, similar to that of the end cap 154. Where the consumable containing package 102 includes a channel through which the consumable aerosol travels, and that channel leads directly to the mouthpiece 158 that is also part of the consumable containing package 102, and the channel is isolated from the receiver 151 and/or the case 202, the receiver 151 and/or the case 202 will remain free of any residue or byproducts formed during operation of the device. In this configuration, the receiver 151 and/or the case 202 stay clean and does not require the user to periodically clean out the receiver 151 and/or the case 202.

## Induction Heating

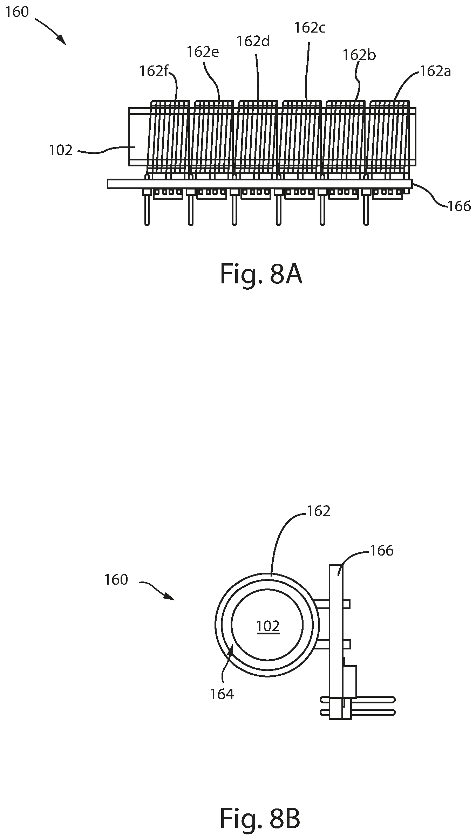

Heating the consumable-containing unit 104 is achieved by an induction heating process that provides non-contact heating of a metal, preferably ferrous metal, by placing the metal in the presence of a varying magnetic field generated by an inductive heating element 160, as shown in Figures 8A-8B. In the preferred embodiment, inductive heating element 160 is a conductor 162 wrapped around into a coil that generates the magnetic field when current is passed through the coil. The metal susceptor 106 is placed close enough to the conductor 162 so as to be within the magnetic field. In the preferred embodiment, the conductor 162 is wrapped in a manner that defines a central cavity 164. This allows the consumable-containing package 102 to be inserted into the cavity 164 to have the conductor 162 surround the susceptor 106 without touching the susceptor 106. The current passed through the conductor 162 is alternating current creating a rapidly alternating magnetic field. The alternating magnetic field may create eddy currents in the susceptor 106, which may generate heat within the susceptor 106. Thus, the consumable-containing package 102 is generally heated from the inside out. In embodiments in which the encasement 108 also serves as the susceptor, the consumable-containing package 102 is heated from the outside in. In some embodiments, a plurality of discrete conductors 162a-f may be serially arranged to allow discrete units of the consumable-containing package 102 to be heated.

In a preferred embodiment, the heating is very rapid and of short duration. It takes approximately less than ten seconds for the device to "warm up" and be ready to deliver the power to the coil, which then heats the susceptor almost immediately to provide the consumable aerosol. Preferably, the warm up time is from about 1 second to about 7 seconds, or from about 3 seconds to about 6 seconds. Most preferably, the warm up time is approximately 2 to 3 seconds. The intense heating of short duration heats the consumable-containing unit 104 from the inside-out, and preferably does not raise the exterior of the consumable-containing unit 104 or the encasement 108 (if so equipped) to combustion temperature. For example, the duration of the intense heating may be from approximately 0.5 to approximately 5 seconds. Preferably, the heating is from approximately 0.5 to approximately 3 seconds. Most preferably, the heating is from approximately 1 to approximately 2 seconds. The heating is sufficient to create a consumable aerosol to be expelled from the consumable-containing unit 104.

In the preferred embodiment, segments of the consumable-containing package 102 are to be heated individually. As such, the conductor 162 may also be provided as individual sets of coiled conductors 162a-f, as shown in Figure 8A. Each conductor coil 162a-f may be attached to a controller 166 that can be controlled to activate one conductor coil 162a-f at a time. Although there are six (6) conductor coils 162a-f shown in Figure 8A, greater or fewer coils could be used. In an alternative embodiment, a single conductor coil 162 may be used, with a mechanical mechanism that translates the coil along the consumable-containing package 102 to individually heat each segment of the consumable-containing package 102.

The individual conductor coils 162a-f may match up with discrete segments of the consumable-containing package 102, as described above, and shown in Figures 3A-6B. Alternatively, the conductor coils 162a-f could each correspond to a certain length of a continuous consumable-containing package 102 such as shown in Figures 2A-2D, 7A, and 7D, to heat only that certain length. In preliminary testing of such embodiments, heating along discrete lengths of the consumable-containing package 102 does not appreciably heat adjacent portions of the consumable-containing package 102, as the adjacent non-heated consumable appears to act as an insulator. Thus, structures to limit heat transfer may not be necessary, although such structures have been discussed herein and may be useful.

The efficiency of conversion of electric power into thermal heat in the susceptor 106 is referred to herein as the "conversion efficiency," and is based on a variety of factors, such as bulk resistivity of the metal, dielectric of the metal, metal geometry and heat loss, power supply consistency and efficiency, coil geometry, and losses and overall frequency of operation-to identify some of these factors. The device 100 is designed and configured to maximize the conversion efficiency.

To further improve the efficiency of the heating process, a pre-heating technique can be used. Preferably, the susceptor 106 is pre-heated to a moderate temperature, as measured by a temperature sensor , then when the user is ready to inhale, the susceptor 106 receives a boost of energy raising the temperature quickly to a higher aerosolizing temperature. For example, when a consumable-containing package 102 is inserted into the device 100, it may trigger the actuation of the heating system 160, which heats the susceptor 160 to about a moderate temperature (i.e. above room temperature, but below an aerosolizing temperature), for example, 200 degrees C. Then, when the user begins sucking on the mouthpiece, the pressure differential created inside the consumable-containing package 102 is detected by a pressure sensor 426, and a burst of energy is generated by the system controller 166 to rapidly increase the temperature to a higher, aerosolizing temperature, for example, 350 degrees C.

In some embodiments, an external actuator can be used wherein when the user is getting ready to inhale, the user can actuate an external actuator to begin the pre-heating process to a moderate temperature. Then, when the user is ready to inhale, a second external actuator can be actuated, or the vacuum created inside by the sucking action can be detected by a pressure sensor 426 or other device that can signal the heating system 160 to generate the burst of energy to rapidly increase the temperature to the aerosolizing temperature. After the user is finished inhaling the vapor, the temperature can drop back down to the moderate temperature, or an intermediate temperature in between the moderate temperature and the aerosolizing temperature. Therefore, when the user is ready to take the next dose, the heating system is primed.

For example, when the consumable-containing package 102 is first engaged in the aerosol producing device 200, the aerosol producing device 200 heats the susceptor anywhere from about 100 degrees C to about 250 degrees C depending on the active ingredient. Then when a puff is taken, the aerosol producing device 200 boosts the temperature to about 400 degrees C or above to create a puff. This boost can be as fast as about 0.01 second. Once a puff has been taken, the aerosol producing device 200 starts to extrapolate the temperature drop via a time curve to calculate the temperature before the next puff. Because the aerosol producing device 200 has a thermostat on one of the chips the starting temperature of the device environment is known. By extrapolating the temperature over time curve, depending on when the next puff is taken, the aerosol producing device 200 calculates exactly how much power is needed to achieve the 400 degrees C on the next puff. This is done again and again after each puff. So if a puff is taken within 5 seconds of the first puff, or 30 seconds after the first puff, the fuzzy logic process will bring the next puff to the 400 degree C temperature (or any set temperature) of the second/next puff. The system controller 166 may be operatively connected to a temperature sensor to measure the temperatures. This system makes for puff consistency that will be similar to moving the coil to get a consistent puff. Therefore, this feature can be the preferred embodiment where the heating coil does not move.

As such, a preferred embodiment for a method for aerosolizing a consumable by an aerosol producing device 200 can comprise preheating a susceptor 106 surrounded by the consumable to a moderate temperature that is above room temperature, but below an aerosolizing temperature; and heating the susceptor 106 from the moderate temperature to the aerosolizing temperature, whereby the consumable is aerosolized. After heating the susceptor 106 to the aerosolizing temperature, the susceptor can be brought back down to the moderate temperature. Alternatively, after heating the susceptor 106 to the aerosolizing temperature, the susceptor 106 can be brought back down to an intermediate temperature that is lower than the aerosolizing temperature, but higher than the moderate temperature. Preferably, preheating the susceptor 106 occurs upon insertion of a consumable-containing package 102 into the aerosol producing device 200. In addition, heating the susceptor 106 to the aerosolizing temperature occurs upon when a pressure sensor 426 detects a pressure drop inside the aerosol producing device 200, such as in the receiver 151.

## Aerosol Producing Device

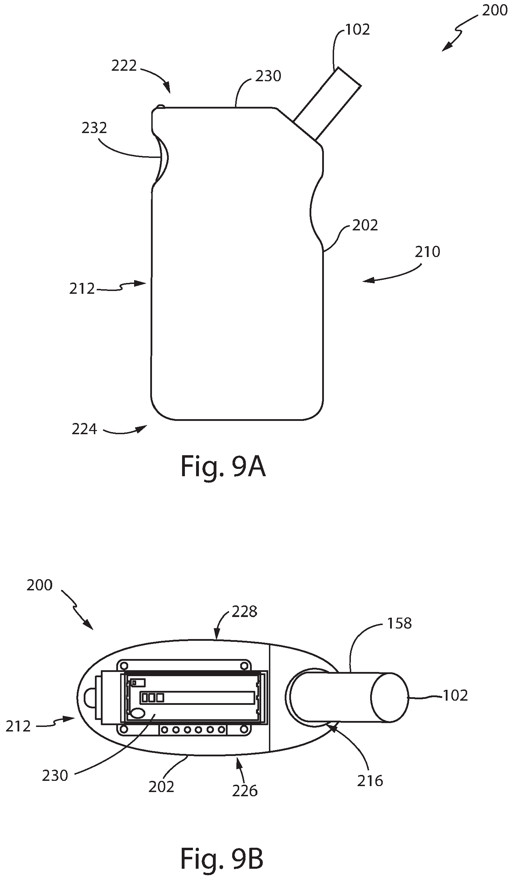

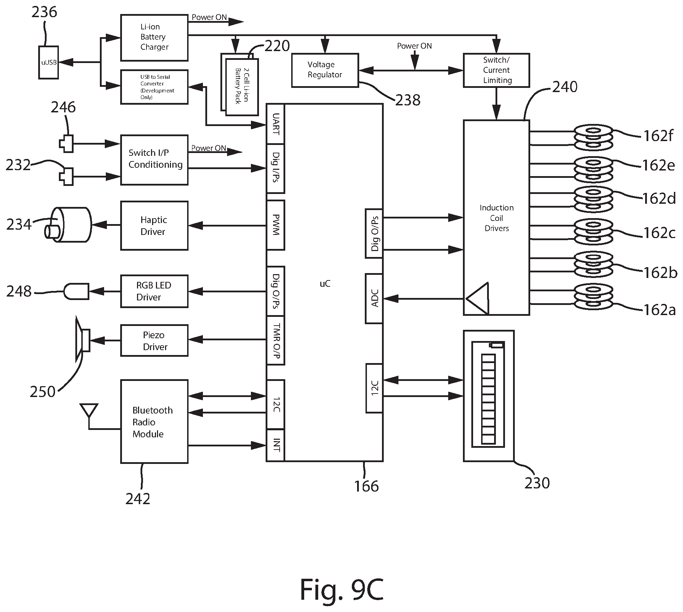

To effectuate the heating and conversion to an aerosol of the consumable, the consumable-containing package 102 is placed inside an aerosol producing device 200, as shown in Figures 1, and 9A-9C. The aerosol producing device 200 comprises a receiver 151 to contain the consumable-containing package 102, the induction heating element 160 to heat the susceptor 106, and a system controller 166 to control the induction heating element 160. The case 202 is designed for ergonomic use. For ease of nomenclature, the case 202 is described using terms such as front, back, sides, top and bottom. These terms are not meant to be limiting, but rather, used to describe the positions of various components relative to each other. For purposes of describing the present invention, the front 210 will be the portion of the case 202 that faces the user when used as intended as described herein. As intended, when the user grasps the case 202 for use, the fingers of the user will wrap around the back 212 of the device 100 with the thumb wrapping around the front 210. The case 202 defines a cavity 214 (see Figure 1) in which the components of the device 100 are contained. As such, the case 202 is designed to contain a substantial portion of the consumable-containing package 102, the controller 166, the inductive heating element 160, and the power source 220. In the preferred embodiment, the top-front portion of the case 202 defines an orifice 216. The mouthpiece portion 158 of the consumable-containing package 102 projects out from the orifice 216 so that the user has access to the consumable-containing package 102. The mouthpiece 158 projects sufficiently out of the case 202 to allow the user to place his or her lips around the mouthpiece 158 to inhale the consumable aerosol.

The case 202 is intended to be user-friendly and easily carried. In the preferred embodiment, the case 202 may have dimensions of approximately 85 mm tall (3.35 inch) (measured from top 222 to bottom 224) by 44 mm (1.73 inch) deep (measured from front 210 to back 212) by 22 mm wide (0.87 inch) (measured from side 226 to side 228). This may be manufactured by proto-molding for higher quality/sturdier plastic parts.

In some embodiments, the consumable-containing package 102 may be held in a retractor that allows the consumable-containing package 102 to be retracted inside the case 202 for storage and travel. Due to the configuration of the consumable-containing package 102, the receiver 151 and/or the case 202 does not need a clean-out through-hole like other devices in which some combustion is still prevalent creating byproduct residue from the combustion. In embodiments where the consumable-containing package 102 comprises a user mouthpiece 158 and filter tube 140, if there are any byproducts created during operation they will remain in the disposable consumable-containing package 102, which is changed out when the user inserts a new consumable-containing package 102, and filter tube 140 if necessary, into the receiver 151 and/or the case 202. Thus, the interior of the receiver 151 and/or the case 202 stays clean during operation.

In the preferred embodiment, the top 222 of the case 202 comprises a user interface 230. Placing the user interface 230 at the top 222 of the case 202 allows the user to easily check the status of the device 100 prior to use. The user could potentially view the user interface 230 even while inhaling. The user interface 230 may be multi-color LED (RGB) display for device status indication during use. A light-pipe may be used to provide wide angle visibility of this display. By way of example only, user interface 230 has a 0.96 inch (diagonal) OLED display with 128x32 format and I2C (or SPI) interface. The user interface 230 is capable of haptic feedback 234 (vibration) and audio feedback 250 (piezo-electric transducer). In some embodiments, a clear plastic (PC or ABS) cover may be placed over the OLED glass to protect it from damage/scratches.

The back 212 of the case comprises a trigger 232, which is a finger activated (squeeze) button to turn the device on/initiate "puff." Preferably, the trigger 232 is adjacent to the top 212. In this configuration, the user can hold the case 202 as intended with his or her index finger on or near the trigger 232 for convenient actuation. In some embodiments, a locking mechanism may be provided on the trigger 232 - either mechanically or through electrical interlock that requires the case 202 to be opened before the trigger 232 is electrically enabled. In some embodiments, a haptic feedback motor 234 may be mechanically coupled to the trigger 232 to improve recognition of haptic feedback by the user during operation. Actuation of the trigger 232 powers the induction heating element 160 to heat the susceptor 106.

The device 100 is powered by a battery 220. Preferably, the battery 220 is a dual cell Li-ion battery pack (series connected) with 4A continuous draw capability, and 650-750mAh rated. The dual cell pack may include protection circuit. The battery 220 can be charged with a USB Type "C" connector 236. The USB type "C" connector 236 can also be used for communications. The controller 166 may also provide for battery voltage monitoring 238 for battery state of charge/-discharge display.

The trigger 232 is operatively connected to the induction coil driver 240 via the controller 166. The induction coil driver 240 activates the inductive heating element 160 to heat the susceptor 106. The present invention eliminates the motor driven coil design in the prior art. The induction coil driver 240 can provide drive/multiplexing for multiple coils. For example, the induction coil driver 240 may provide drive/multiplexing for 6 or more coils. Each coil is wrapped around one segment of the consumable-containing package 102 and can be actuated at least one or more times. Therefore, one segment of the consumable-containing package 102 can be heated twice, for example. In a device 100 having six coils, the user could extract 12 "puffs" from the device 100.

The induction coil drive circuit in the preferred embodiment may be directly controlled by a microprocessor controller 166. A special peripheral in this processor (Numerically Controlled Oscillator) allows it to generate the frequency drive waveforms with minimal CPU processing overhead. The induction coil circuit may have one or more parallel connected capacitors, making it a parallel resonant circuit.

The drive circuit may include current monitoring with a "peak detector" that feeds back to an analog input on the processor. The function of the peak detector is to capture the maximum current value for any voltage cycle of the drive circuit providing a stable output voltage for conversion by an analog-to-digital converter (part of the microprocessor chip) and then used in the induction coil drive algorithm.

The induction coil drive algorithm is implemented in firmware running on the microprocessor. The resonant frequency of the induction coil and capacitors will be known with reasonable accuracy by design as follows:

$$\text{Frequency of resonance (in Hertz)} = 1/(2*pi* SQRT\{L*C\})$$

where: pi = 3.1415...,
SQRT indicates the square root of the contents in the brackets {...},
L = the measured inductance of the induction coil, and
C = the known capacitance of the parallel connected capacitors.

There will be manufacturing tolerances to the values of L and C (from above), which will produce some variation in the actual resonant frequency versus that which is calculated using the formula above. Additionally, there will be variation in the inductance of the induction coil based on what is located inside of this coil. In particular, the presence of a ferrous metal inside (or in the immediate vicinity) of this coil will result in some amount of inductance change resulting in a small change in the resonant frequency of the L-C circuit.

The firmware algorithm for driving the induction coil will sweep the frequency of operation over the maximum expected frequency range, while simultaneously monitoring the current, looking for the frequency where the current draw is at a minimum. This minimum value will occur at the frequency of resonance. Once this "center frequency" is found, the algorithm will continue to sweep the frequency by a small amount on either side of the center frequency and adjust the value of the center frequency as required to maintain the minimum current value.

The electronics are connected to the controller 166. The controller 166 allows for a processor based control of frequency to optimize heating of the susceptor 106. The relationship between frequency and temperature seldom correlates in a direct way, owing in large part to the fact that temperature is the result of frequency, duration and the manner in which the consumable-containing package 102 is configured. The controller 166 may also provide for current monitoring to determine power delivery, and peak voltage monitoring across the induction coil to establish resonance. By way of example only, the controller may provide a frequency of approximately 400 kHz to approximately 500 kHz, and preferably, 440 kHz with a three-second pre-heat cycle to bring the temperature of the susceptor 106 to 400 degrees Celsius or higher in one second. In some embodiments, the temperature of the susceptor 106 can be raised to 550 degrees Celsius or higher in one second. In some embodiments, the temperature can be raised as high as 800 degrees Celsius. Thus, the present invention has an effective range of 400-800 degrees Celsius. In prior art devices, such temperatures would combust the consumable, making the prior art devices ineffective at these temperatures. In the present invention, such high temperatures can still be used to improve the efficiency of aerosol production and allow for quicker heat times.

The device 100 may also comprise a communications system 242. In the preferred embodiment, Bluetooth low energy radio may be used to communicate with a peripheral device. The communications system 242 may serial interface to the main processor for communicating information with a phone, for example. Off-the-shelf RF module (pre-certified: FCC, IC, CE, MIC) can also be used. One example utilizes Laird BL652 module because SmartBasic support allows for rapid application development. The communication system 242 allows the user to program the device 100 to suit personal preferences related to the aerosol density, the amount of flavor released, and the like by controlling the frequency and the 3-stage duty cycle, specifically, the pre-heat stage, heating stage, and wind-down stage of the inductive heating elements 160. The communication system 242 may have one or more USB ports 236.

In some embodiments, an RTC (Real-time Clock/Calendar) with battery back-up may be used to monitor usage information. The RTC can measure and store relevant user data to be used in conjunction with an external app downloaded on to a peripheral device, such as a smartphone.

In some embodiments, a micro-USB connector (or USB type C connector or other suitable connector) may be located on the bottom of the case 202. Support connector with plastics may be provided on all sides to reduce stress on connector due to cable forces.

By way of example only, the device 100 may be used as follows. Power for the device may be turned on from momentary actuation of the trigger 232. For example, a short press of the trigger (<1.5 sec) may turn the device 100 on but does not initiate the heating cycle. A second short press of the trigger 232 (<1 sec) during this time will keep the device 100 on for a longer period of time and initiate Bluetooth advertising if no active (bonded) Bluetooth connection with phone currently exists. A longer press of the trigger 232 (>1.5 sec) initiates the heating cycle. The power for the device 100 may remain on for a short period of time after each heating cycle (e.g., 5 sec) to display updated unit status on the OLED user interface 230 before powering off. In some embodiments, the device 100 may power on when the consumable-containing package 102 is deployed from the case 202. In some embodiments, a separate power switch 246 may be used to turn the device on and off.

When an active connection is found with a smartphone and the custom application is running on the smartphone, then the device 100 will remain powered on for up to 2 minutes before powering off. When the battery level is too low to operate, the user interface display 230 flashes several times (showing battery icon at "0%" level) before turning unit off.

In some embodiments, the user interface 230 may display a segmented cigarette showing which segments remain (solid fill) versus which segments have been used (dotted outline) as an indicator of how much of the consumable-containing package 102 still contains consumable products to be released. The user interface 230 can also display a battery icon updated with current battery status, charging icon (lightning bolt) when the device is plugged in, and a Bluetooth icon when active connection exists with a smartphone. The user interface 230 may show the Bluetooth icon flashing slowly when no connection exists but the device 100 is advertising.

The device may also have an indicator 248 to inform the user of the power status. The indicator 248 may be an RGB LED. By way of example only, the RGB LED can show a green LED on when the device is first powered on, a red LED flashing during the preheat time, a red LED on (solid) during the "inhale" time, and a blue LED flashing during charging. Duty cycle of flashing indicates the battery's relative state of charge (20-100%) in 20% increments (solid blue means fully charged). A fast flashing of blue LED may be presented when an active Bluetooth connection is detected (phone linked to device and custom app on phone is running).

Haptic feedback can provide additional information to the user during use. For example, 2 short pulses can be signaled immediately when power is turned on (from finger trigger button). An extended pulse at the end of preheat cycle can be signaled to indicate the devices refer inhalation (start of HNB "inhale" cycle). A short pulse can be signaled when USB power is first connected or removed. A short pulse can be signaled when an active Bluetooth connection is established with an active phone app running on the smartphone.

A Bluetooth connection can be initiated after power is turned on from a short (<1.5 sec) press of the finger grip button. If no "bonded" BLE (Bluetooth Low Energy) connection exists, that the devices may begin slow advertising ("pairing" mode) once a second short press is detected after initial short press is detected that powers the device on. Once a connection is established with the smartphone application, the Bluetooth icon on the user interface display 230 may stop flashing and the blue LED will turn on (solid). If the device 100 is powered on and it has a "bonded" connection with a smartphone, then it may begin advertising to attempt to re-establish this connection with the phone up until it powers off. If the connection with this smartphone is able to be re-established, then the unit may remain powered on for up to 2 minutes before powering itself off. To delete a bonded connection, the user can power the device on with a short press followed by another short press. While BLE icon is flashing, the user can press and hold the trigger 232 until the device 100 vibrates and the Bluetooth icon disappears.

So, by tight control of the afore-mentioned conversion efficiency factors and the product consistency factors, it is possible to provide controlled delivery of heat to the consumable-containing unit 104. This controlled delivery of heat involves a microprocessor controller 166 for the monitoring of the induction heating system 160 to maintain various levels of electrical power delivery to the susceptor 106 over controlled intervals of time. These properties enable a user-control feature that would allow the selection of certain consumable flavors as determined by the temperature at which the consumable aerosol is produced.

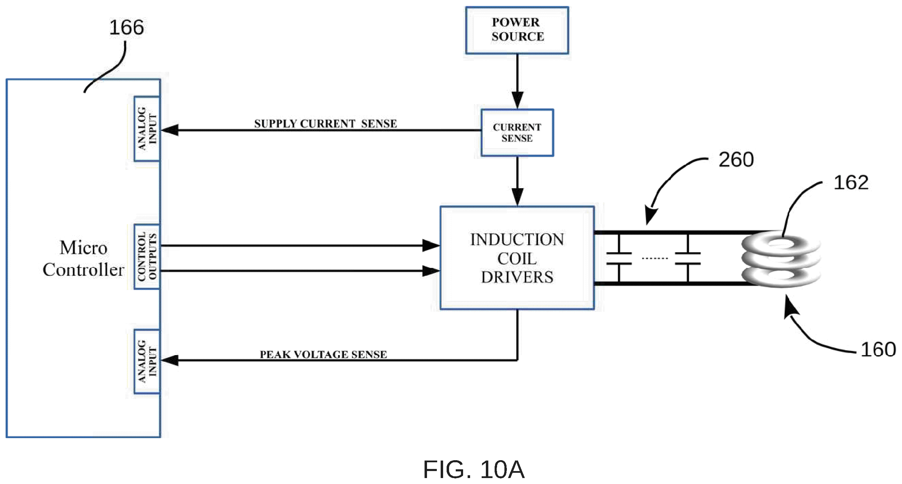

In some embodiments a microprocessor or configurable logic block can be used to control the frequency and power delivery of the induction heating system. As shown in Figure 10A, an induction heating system 160 may comprise a wire coil 162 in parallel with one or more capacitors 260 to and from a self-resonant oscillator. The inductance of the coil 162 in combination with the capacitance of the capacitor(s) 260 largely defines the resonant frequency at which the circuit will operate. In this embodiment, however, a microprocessor/microcontroller 166 can instead be used to drive the power switches and hence control the frequency of oscillation of the circuit. With this approach, the peak voltage and current are used as feedback to allow the microprocessor control program to provide closed tuning to find resonance. The benefit of this approach is that it allows efficient control of the power delivered to the susceptor by synchronously switching the oscillation of the circuit on and off under the control of the microprocessor 166 control program and provides optimal on/off switching of the power control elements driving the induction coil system.

Based on these concepts, a number of variations have been contemplated by the inventors. Thus, as discussed above, the present invention comprises a consumable-containing unit 104, a susceptor 106 embedded within the consumable-containing unit 104, a heating element 160 configured to at least partially surround the consumable-containing unit 104, a controller 166 to control the heating element 160, and a receiver 151 and/or a case 202 to contain the consumable-containing unit 104, the susceptor 106, the heating element 160, and the controller 166. Preferably, the consumable-containing unit 104 is contained with the susceptor 106 in a consumable-containing package 102. As such, any description of the relationships between the consumable-containing package 102 with other components of the invention may also apply to the consumable-containing unit 104, as some embodiments may not necessarily require packaging of the consumable-containing unit 104.

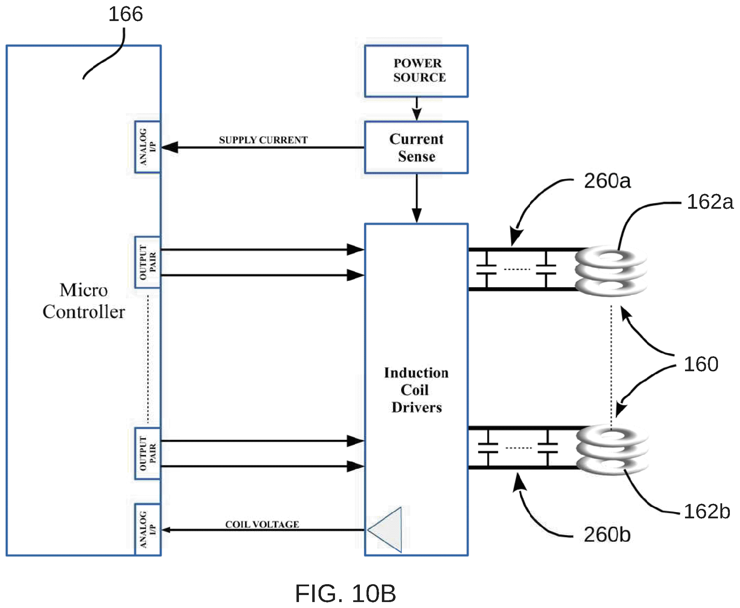

In some embodiments, as shown in Figure 10A, the device comprises a self-resonant oscillator for controlling the inductive heating element 160. The self-resonant oscillator comprises a capacitor 260 operatively connected to the inductive heating element 160 in parallel. In some embodiments, as shown in Figure 10B, multiple heating elements 160 may be connected in parallel with their respective capacitors 260a, 260b. Preferably, the heating elements are in the form of a coiled wire 162a, 162b.

To allow a single consumable-containing package 102 to generate aerosol multiple times, multiple heating elements 160 and/or moveable heating elements 160 may be used. Thus, the heating element 160 comprises a plurality of coiled wires 162a, b, where each coiled wire may be operatively connected to the controller 166 for activation independent of the other coiled wires.

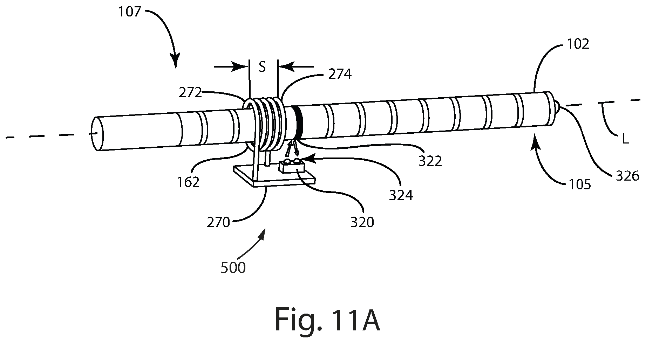

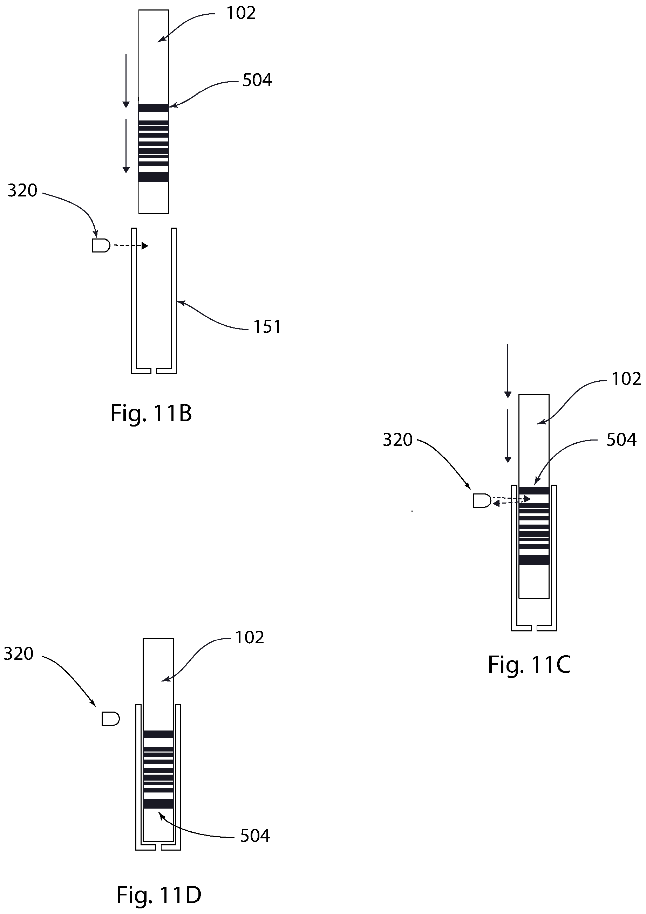

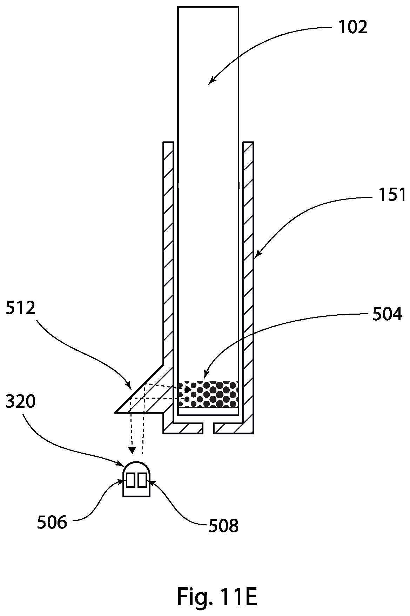

In some embodiments, the heating element 160 may be moveable. In such embodiments, the consumable-containing package 102 may be an elongated member defining a first longitudinal axis L, and the heating element may 162 be configured to move axially along the first longitudinal axis L. For example, as shown in Figure 11A-E, the heating element 160 may be attached to a carrier 270. The carrier 270 may be operatively connected to the receiver 151 so as to move along the length of the consumable-containing package 102 while the heating element 160 remains coiled around the consumable-containing package 102. The span S of the coil (measured as the linear distance from the first turn 272 of the coil to the last turn of the coil 274) may be short enough only to cover a segment of the consumable-containing package 102. Once the heating element 160 has been activated at that segment, the carrier 270 advances along the consumable-containing package 102 along its longitudinal axis L to another segment of the consumable-containing package 102. The distance of travel of the carrier 270 is such that the first turn 272 of the coil stops adjacent to where the last turn 274 of the coil had previously resided. Thus, a new segment of equal size to the previously heated segment is ready to be heated. This can continue until the carrier 270 moves from the first end 105 of the consumable-containing package 102 to the opposite end 107. Alternatively, the coil could be stationary and the consumable-containing package 102 could be made to move to accomplish a similar heating of segments. In such an embodiment, the receiver 151 may have to be of increased length to accommodate the movement of the consumable-containing package 102, and a carrier 270 would have to be adapted to move the consumable-containing package 102 rather than the coil.

In embodiments in which the consumable-containing package 102 contains multiple consumable-containing units 104, the span S of the coil, may be approximately the same size as the length of the consumable-containing unit 104.

The carrier 270 may be configured to align the coil with a consumable-containing unit 104 so that the coil can heat an entire consumable-containing unit 104. The carrier 270 may be configured to move the coil from one consumable-containing unit 104 to the next, again allowing a single consumable-containing package 102 to be heated multiple times with the aerosol being released each time.

**Recognition System**

In some embodiments, it is desirable to be able to detect whether a consumable-containing unit 104, or a portion thereof, has been heated or not. If a consumable-containing unit 104 has already been heated, then the heating element 160 can heat the next consumable-containing unit 104 or the next segment of a consumable-containing unit 104 so as to prevent energy from being wasted on a used portion of the consumable-containing unit 104. Therefore, in some embodiments, as shown in Figure 11A, a recognition system 500 is used to detect the segments of the consumable-containing package 102 that have been used is provided in the device, allowing the device to autonomously determine the next unused segment that is available for use. For example, the device may comprise an optical sensor 320 to detect whether a portion of the consumable-containing package 102 being sensed had been heated beyond a predetermined temperature. In some embodiments, the optical sensor 320 may detect visual changes in the consumable-containing package 102 that is indicative of heating. In some embodiments, the optical sensor 320 may detect thermal changes in the consumable-containing package 102 that is indicative of heating. In some embodiments, the optical sensor 320 may detect textural changes (i.e. changes in the texture) in the consumable-containing package 102 that is indicative of heating. In some embodiments, the optical sensor 320 may be the controller keeping track of where the heating element 160 is along the consumable-containing package 102 and when it has been heated relative to its movement along the consumable-containing package 102. For example, the controller may comprise a memory for storing locations of the portions of the consumable-containing package 102 that have been heated to the predetermined temperature.

In the preferred embodiment, the optical sensor 320 is a photoreflective sensor. The photoreflective sensor may be configured to detect changes in the consumable-containing package 102 from its original state compared to a state when the consumable-containing package 102 has been exposed to significant heat (i.e. beyond normal temperatures of the day). More preferably, the consumable-containing package 102 may be comprised of a thermal sensitive dye that changes colors when heated to a predetermined temperature. Such change in color may be detectable by the photoreflective sensor. The thermally sensitive dye may be printed around the exterior surface of the consumable-containing package 102. When a segment of the consumable-containing package 102 is heated, a band 322 in closest proximity to the heated segment changes colors. For example, the band 322 may change from white to black. The optical sensor 320 mounted with the heating element 160 has optics 324 focused just above-or below-the heating element to provide a side view of the consumable-containing package 102 over the full range of the moving heating element 160.

In some embodiments, a limit switch 326 is also installed at one end 105 of the consumable-containing package 102 and used to detect when the consumable-containing package 102 is removed or reinserted into the device. When a consumable-containing package 102 has been re-inserted, the device activates the motorized heating element assembly and moves it across its full range of travel, allowing the optical sensor 320 to detect if any segments have been previously heated, by detecting the dark bands 322 of the thermally sensitive dye. Thus, the device may further comprise a limit switch 326 to reset the memory when a new consumable-containing package 102 is inserted into the housing.

Furthermore, the recognition system 500 utilizes sensing technology to identify the presence, type, and other information about the consumable so as to configure the aerosol producing device 200 to execute an administration protocol designed for the consumption of that consumable. The various different consumables that can be used by the present system can have a specific temperature and duration of heat exposure to optimize and release the proper dosage of the consumable. In some situations, there may be limitations in the number of doses a user can take in a set period of time. Therefore, rather than a single temperature and duration that might be optimal for one consumable and not others, or that might be the highest tolerable temperature and duration sufficient for aerosolizing all potential consumables, the recognition system 500 automatically customizes the temperature and duration based on the consumable and any prescription instruction, as well as performing other features, such as determining whether a consumable is present, whether a consumable has been used up, whether the user has exceeded the number of uses in set period of time, and the like.

For example, with reference to Figures 11B-11E, in some embodiments, the recognition system 500 may comprise an optical sensor 320. The optical sensor 320 can be configured to read a sign 504 on the consumable-containing package 102 and correlate that sign 504 with a set of instructions related to how the aerosol producing device 200 should behave with respect to the consumable contained in the consumable-containing package 102. For example, the sign 504 can be a specific pattern as seen with bar codes, QR codes, and the like, or a specific spectrum of light, sequence of colors, specific patterning, or any combination thereof. In some embodiments, the optical sensor 320 may comprise a light source 506 covering either a narrow or broad portion of the visible or invisible (ultra-violet or infra-red) light spectrum to illuminate at least one portion of the consumable-containing package 102. The optical sensor 320 may further comprise a light sensor 508 for the purpose of sensing the reflected light/light spectrum from this portion of the consumable-containing package 102.

A light sensor 508 that is sensitive in the same spectrum range as the light source 506, positioned to optimally detect the reflected light from the light source 506 off of the perimeter of the consumable-containing package. The light sensor 508 may be either capable of detecting light intensity at one or more ranges of the visible or invisible light spectrum, or it may be able to detect both light intensity and the wavelengths (color) of the reflected light.

In some embodiments, the sign 504 can be one or more bands of colored, grayscale, and/or invisible (UV sensitive) ink printed on the consumable-containing package 102, and preferably, around the perimeter of the consumable-containing package 102. Preferably, the sign 504 is near the first end 152 of the consumable-containing package 102 so that the sign 504 can be detected when the consumable-containing package 102 is fully inserted into the aerosol producing device 200 or while the consumable-containing package 102 is in the process of being inserted into the aerosol producing device 200.

Electronic circuitry and a system controller 166 located in the aerosol producing device 200 controls the light source 506 and monitors the light sensor 508 for the presence of the consumable-containing package 102, and processes the data received by the light sensor 508 for the purpose of identifying one or more characteristics of this consumable-containing package 102 so as to keep within the proper administration protocol.

Thus, the light sensor 508 may be able to read the sign as the consumable-containing package 102 is being inserted into the aerosol producing device 200 or after it has been properly seated.

In some embodiments, the recognition system 500 may further comprise optical modifiers 512 to guide, bend, focus, and select the desired light to a desired area. For example, optical modifiers 512 can be optical waveguides (light-pipes), reflective surfaces, light apertures, prisms, lenses, filters, polarizers, beam splitters, fiber optics, and the like. Similarly, there may be optical modifiers 512 positioned to direct the light reflected from the consumable-containing package 102 back to active area on the light sensor 508.

Uses of different types of light sources 506 and light sensors 508 allows for multiple modes of detection. For instance, an invisible UV light source/detector could be used in conjunction with a color light source/detector allowing the device to potentially detect a counterfeit versus legitimate product being used. In other words, because a counterfeiter would not have seen the invisible light, the counterfeiter would not have put such a band 510 on the counterfeit consumable-containing package 102, and the device 100 would not work.

By way of example only, the light source 506 may emit all spectrum of visible light and some invisible light such as infrared and ultraviolet. One consumable-containing package 102 may have a band 510 that reflects light in the blue spectrum. As such, the light sensor 508 detects a blue color. A blue color may correspond with the consumable-containing package containing nicotine. As such, the aerosol containing device 200 will configure the heating system to heat to the proper temperature for an appropriate duration of time to release nicotine from the consumable-containing package 102. If on the other hand, the consumable-containing package 102 contained a band 510 that reflects the red spectrum, the light sensor detects a red color. A red color may correspond with the consumable-containing package containing cannabis. As such, the aerosol containing device 200 will configure the heating system to heat to the proper temperature for an appropriate duration of time to release cannabis from the consumable-containing package 102. Many different colors and combination of colors, or patterns can be used to code for many different consumables.

Additionally, the recognition system 500 can provide a notice feature. For example, the color of the consumable-containing package 102 can change after it has been used for a specifically programmed number of puffs, indicating when the consumable has been fully consumed. Such an indication would then render the consumable-containing package 102 unable to be used in the device any longer.

Furthermore, the recognition system 500 can be used to trigger a disabling response to render the consumable unusable. For example, a burst of power can be produced that causes a hole in the consumable-containing package 103 that renders it unable to be used any longer.

Therefore, in a preferred embodiment, a device 100 for generating aerosol may comprise a consumable-containing package 102 comprising a consumable, a susceptor 106 surrounding the consumable, and a sign 504; an aerosol producing device 200 comprising a heating element 160 to inductively heat the susceptor 106, and a recognition system 500 configured to read the sign 504; and a system controller 166 operatively connected to the recognition system 500 and the heating element 160, wherein information received from the recognition system 500 is used to control the heating element 160. The recognition system 500 comprises an optical sensor 320. The optical sensor 320 comprises a light sensor 508 to detect light, specifically, a wavelength of light, reflected off of the sign 504. The optical sensor 320 can further comprises a light source 506 configured to emit a spectrum of light towards the sign 504. The system controller 166 can then execute an administration protocol specific to the consumable associated with the sign 504.

In use, then a method of executing an administration protocol for aerosolizing a consumable, comprises reading a sign 504 on a consumable-containing package 102 containing the consumable with an optical sensor 320; identifying the administration protocol for the consumable associated with the sign 504 with a system controller 166; executing the administration protocol, whereby the consumable is aerosolized. The method can further comprise detecting a pattern on the sign 504, or detecting a wavelength of light reflected from the sign 504 using the light sensor 508. The method can further comprise a light source 106 emitting a spectrum of light towards the sign 504 in order for the sign 504 to reflect a certain wavelength of light back to the sensor 508. The method can further comprise the system controller 166 executing a disabling response to render the consumable unusable.

**Aligner**

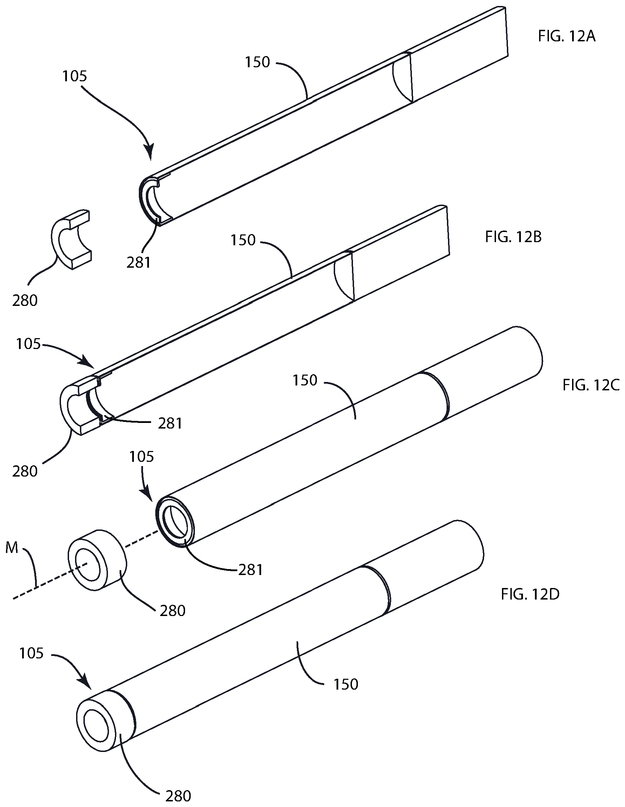

As shown in Figures 12A-12E, to facilitate proper alignment of the heating element 160 around the consumable-containing package 102, the device 200 may comprise a package aligner. For example, the package aligner may be a magnet 280. Preferably, the magnet 280 is a cylindrical magnet defining a second longitudinal axis M. In embodiments in which the heating element 160 is a cylindrical coil wrapped around the consumable-containing package 102, the cylindrical coil defines a third longitudinal axis C. The cylindrical magnet 280 and the heating element 160 are configured to maintain collinear alignment of the second longitudinal axis M with the third longitudinal axis C. Preferably, the cylindrical magnet 280 is a round ring magnet, where the center is a path for air flow. Preferably, any magnet 280 would be a rare earth neodymium type. It would be axially magnetized.

In the embodiment using a magnet 280 for alignment, one end 105 of the consumable-containing package 102 may comprise a magnetically attractive element 281. Preferably, the magnetically attractive element 281 is a stamped ferrous sheet metal component that is manufactured into the first end 105 of the consumable-containing package 102. The cylindrical magnet 280 could be part of the aerosol producing device 200 and the consumable-containing package 102 could have a magnetically attractive element 281 or washer attached to its end 105 so that the consumable-containing package 102 is pulled onto the magnet 280 affixed to the aerosol producing device 200. Other combinations of magnets 280 and magnetically-attractive elements 281, in various positions, may be used to accomplish the desired alignment.

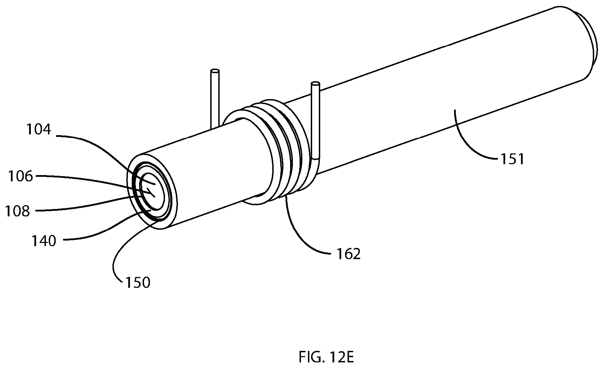

In some embodiments, preferably one that uses a consumable-containing package 102 with a filter tube 140 and a housing 150, the package aligner may be a receiver 151, such as a closely-fitting cylinder (if the housing 150 is cylindrical) that may be used to align the consumable-containing package 102, and the coil 162 could be positioned outside the receiver 151, as shown in Figure 12E. The receiver 151 can be fixed to the device 200 and aligned properly with the coils 162 such than when the consumable-containing package 102 is inserted into the coils 162, the susceptor 106 is properly aligned with the coils 162.

In some embodiments, the housing 150 may function as the receiver 151. Therefore, rather than a separate receiver 151, the housing 150 may have the characteristics described above and insertion into the coils 162 may function as the alignment process, or the housing can be fixed within the coils 162 and the filter tube 140 containing the consumable-containing unit 104 and the susceptor 106 can be inserted into the housing 150.

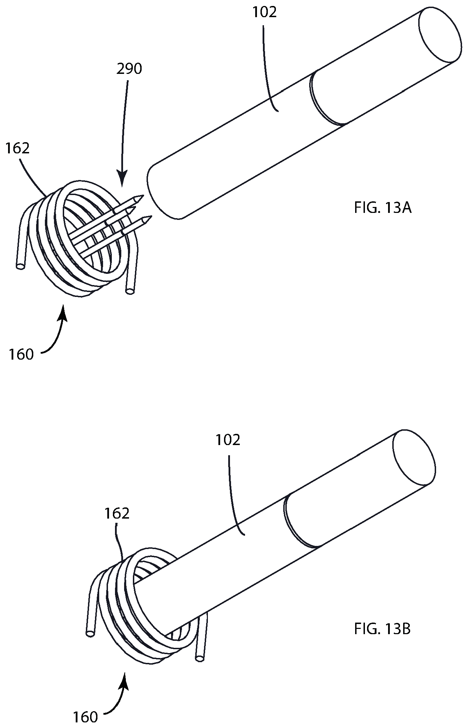

In some embodiments, multiple activations of a single consumable-containing package can be accomplished with a susceptor 106 having multiple prongs 290 as shown in Figures 13A-D. A multi-pronged susceptor is a susceptor 106 with two or more prongs 290. In some embodiments, the susceptor may have three prongs 290a, 290b, 290c. In some embodiments, the susceptor 106 may have four prongs. In some embodiments, the susceptor 106 may have more than four prongs. In the preferred embodiment, the multi-pronged susceptor 106 has three or four prongs.

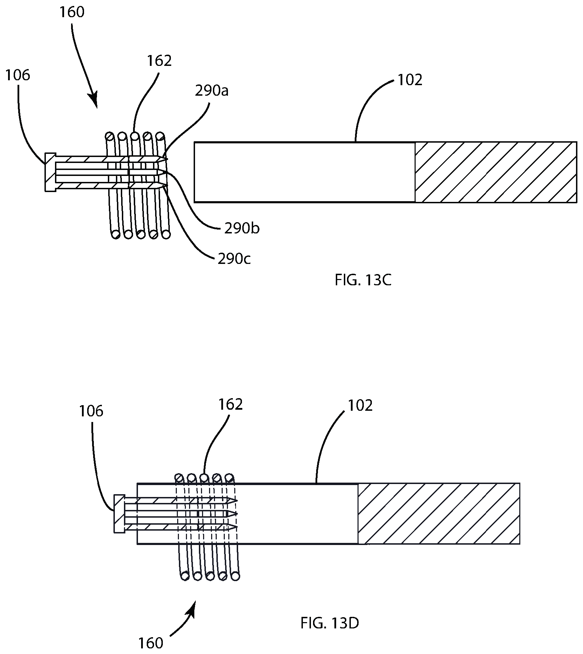

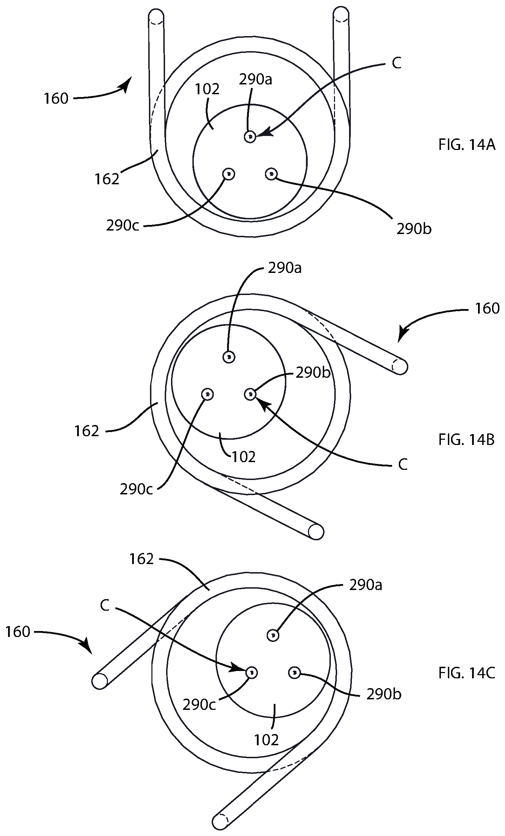

The multiple prongs 290a, 290b, 290c of the multi-pronged susceptor 106 are generally parallel to each other as shown in Figures 13C and 13D. The multi-pronged susceptor 106 is configured and may be embedded into the consumable-containing package 102 in such a way that each prong 290a, 290b, 290c is parallel to and equally spaced from the longitudinal axis of the consumable-containing package L, and equally spaced apart from each other along the perimeter of an imaginary circle. As such, when viewed in cross-section, as shown in Figures 14A-C, the susceptor prongs 290a, 290b, 290c are equally spaced apart from each other about the circular face of the consumable-containing package 102. Such arrangement allows each prong 290a, 290b, 290c to maximize non-overlapping heating zones for each prong, when each prong is maximally activated. In other words, when a susceptor prong 290a, 290b, 290c is heated, it will radiate heat radially away from the susceptor prong 290a, 290b, 290c creating a circular heating zone with the susceptor prong 290a, 290b, 290c in the center. Each susceptor prong 290a, 290b, 290c will heat its own circular zone, although some overlap may be inevitable. Collectively, an entire cross-sectional area of a consumable-containing unit 104 can be heated, one cross-sectional segment at a time.

When the heating element 160 is a cylindrical coil wrapped around a susceptor 106, the maximum amount of energy is transferred to the center of the cylindrical coil. Therefore, when the susceptor 106 is aligned with the center of the cylindrical coil, the susceptor 106 will receive the maximum amount of energy from the electricity passing through the coil. In other words, when the susceptor prong 290a, 290b, 290c is collinear with the cylindrical coil, the susceptor prong 290a, 290b, 290c will receive the maximum amount of energy from the cylindrical coil. Thus, to heat each susceptor prong 290a, 290b, 290c independently, the susceptor prong 290a, 290b, 290c and the center of the coil must be moved relative to each other so that the center of the coil aligns with one of the susceptor prongs 290a, 290b, 290c in sequence. This can be accomplished by moving the susceptor prong relative to the coil, or by moving the coil relative to the susceptor prong, or both.

**Moving Heating Element**

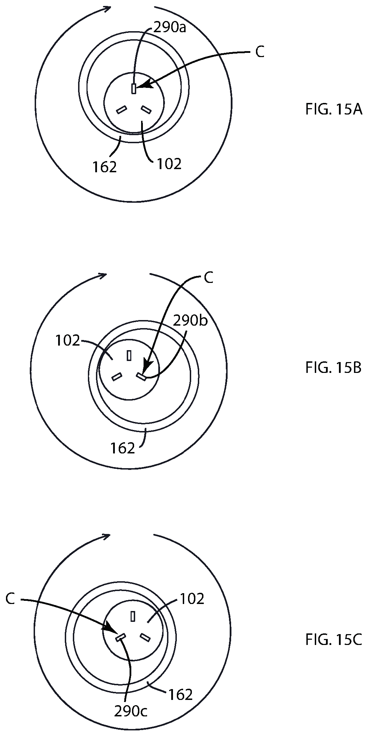

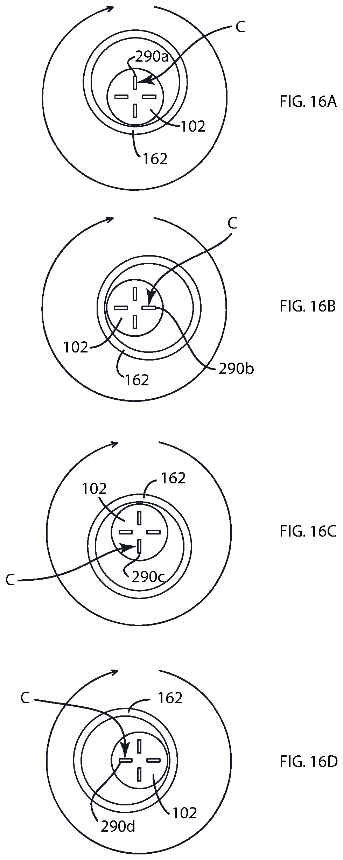

In the preferred embodiment, the heating element 160 moves relative to the susceptor 106. For example, the cylindrical coil may be wrapped around the consumable-containing package 102 and configured to rotate along an eccentric path so that during one rotation of the cylindrical coil each of the prongs 290a, 290b, 290c will align with the center of the coil at different times as shown in Figures 14A-16D. The consumable-containing package 102 may be an elongated member defining a first longitudinal axis L, wherein the heating element 160 is a coil wrapped around the consumable-containing package 102 to form a cylinder defining a second longitudinal axis C, and wherein the heating element 160 is configured to rotate about the consumable-containing package 102 in an eccentric path such that the second longitudinal axis C aligns collinearly with each of the prongs 290a, 290b, 290c of the multi-pronged susceptor at some point during the movement of the heating element about the consumable-containing package 102. Therefore, the multi-prong susceptor 106 is stationary and the coil moves rotationally in an eccentric path so that coil center aligns with the linear axis of each susceptor prong 290a, 290b, 290c, in turn, through the rotation. Electrical slip rings would provide energy to an eccentric path rotating coil design.

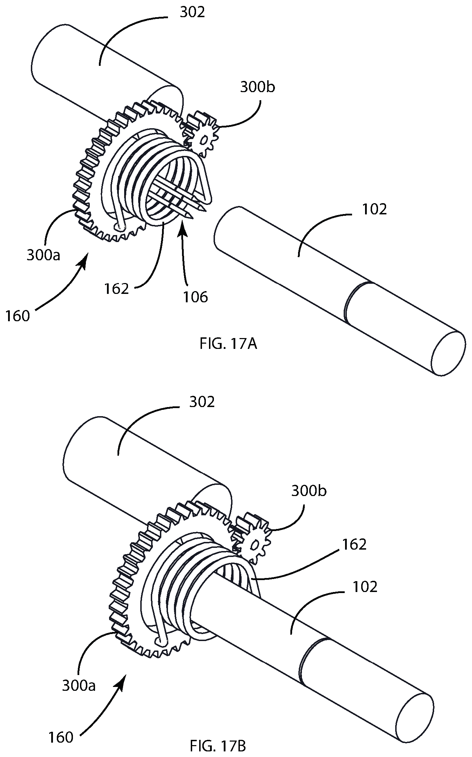

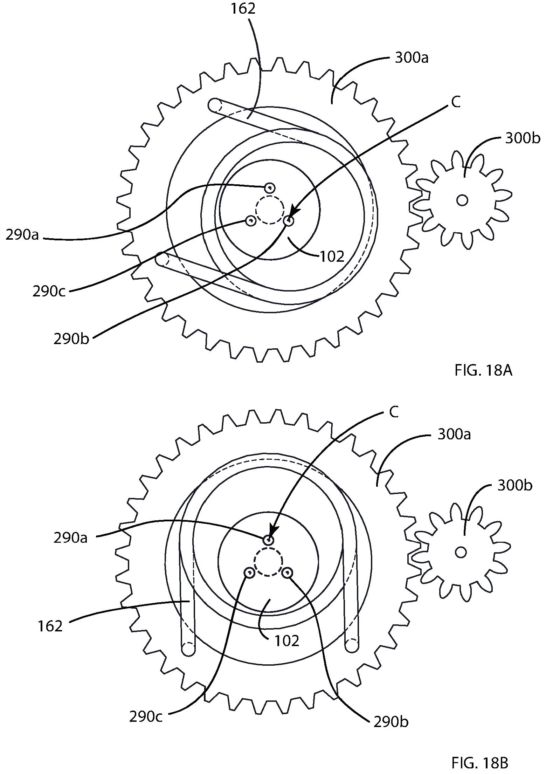

Rotation of the heating element 160 can be effectuated by a series of gears 300a, 300b operatively connected to a motor 302. For example, as shown in Figures 17A-18B, the heating element 160 may be mounted on a first gear 300a so that the heating element can rotate with the first gear 300a. A second gear 300b can be operatively connected to the first gear 300a such that rotation of the second gear 300b causes rotation of the first gear 300a. The second gear 300b may be operatively connected to a motor 302 to cause the second gear 300b to rotate. The heating element 160 is mounted to the first gear 300a in such a manner that rotation of the first gear 300a causes the longitudinal axis C of the heating element 160 to move along an eccentric path rather than causing the heating element to rotate about a fixed, non-moving center. Thus, the center of the heating element 160 can shift to align with the different prongs 290a, 290b, 290c.

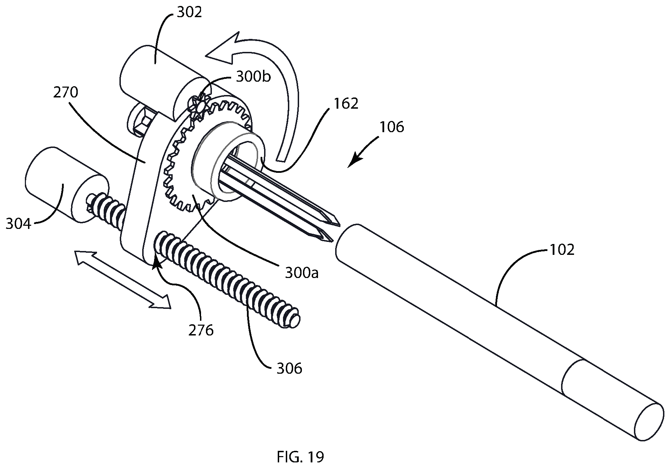

In some embodiments, the heating element 160, the gears 300a, 300b, and the motor 302 may be mounted on a carrier 270 as shown in Figure 19. The carrier 270 allows the heating element, gears 300a, 300b and the motor 302 to move axially along the length of the consumable-containing package 102. The carrier 270 may be operatively connected to a driver 306, which is operatively connected to a second motor 304. For example, the driver 306 may be threaded. The carrier 270 may have a threaded hole 276 through which the driver 306 is inserted. Activation of the second motor 304 causes the driver 306 to rotate. Rotation of the driver 306 causes the carrier 270 to move along the driver 306 as shown by the double arrow in Figure 19.

In some embodiments, rather than having the heating element 160 rotate along an eccentric path, the heating element 160 can be moved translationally along the X-Y axis when viewed in cross section. Therefore, the consumable-containing package 102 may be an elongated member defining a longitudinal axis L, and wherein the heating element 160 is configured to move radially relative to the longitudinal axis L when viewed in cross-section to align the center of the cylindrical, coiled heating element 160 with each of the prongs 290a, 290b, 290c of the multi-pronged susceptor 106, in turn. In the X-Y axis positioning scenario the coil energy could be supplied through a flexible electrical conductor or by moving electrical contacts.

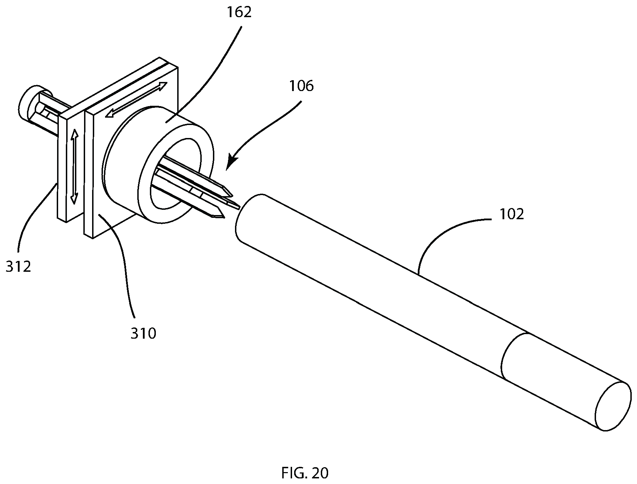

For example, the heating element 160 may be operatively mounted on a pair of translational plates 310, 312 as shown in Figure 20. Specifically, the heating element 160 may be mounted directly on a first translational plate 310, and the first translational plate 310 may be mounted on a second translational plate 312. The first translational plate 310 may be configured to move in the X or Y direction, and the second translational plate 312 may be configured to move in the Y or X direction, respectively. In the example shown in Figure 20, the first translational plate 310 is configured to move in the X direction, while the second translational plate 312 is configured to move in the Y direction. This configuration can be switched so that the first translational plate 310 is configured to move in the Y direction and the second translational plate 312 is configured to move in the X direction. The first and second translational plates 310, 312 may be operatively connected to their respective motors, for example, via gears, to cause the translational plates to move in the appropriate direction. Between the two translational plates 310, 312, the heating element 160 can be moved so that its longitudinal axis C can align collinearly with any of the prongs 290a, 290b, 290c.

In other arrangements the coil assembly could move along the susceptor's linear axis, independent of a rotation or non-rotation movement mechanisms as discussed above. Therefore, a three pronged susceptor would allow the device to heat a consumable-containing package 102 three times at the same linear position by heating the three different prongs 290a, 290b, 290c three different times before it moves to its next linear position, where it will be able to heat three times again. In a consumable-containing package 102 having four linear positions, one consumable-containing package should be able to provide 12 distinct "puffs," i.e. 3 prongs times 4 positions along the length of the consumable-containing package 102.

In some embodiments, rather than having the heating element 160 move relative to the consumable-containing package 102, the consumable-containing package 102 can be moved relative to the heating element. Therefore, the consumable-containing package 102 is configured to rotate within the heating element 160 in an eccentric path such that the second longitudinal axis C defined by the coils aligns collinearly with each of the prongs 290a, 290b, 290c of the multi-pronged susceptor at some point during the rotation of the consumable-containing package 102 within the heating element 160. Alternatively, the consumable-containing package 102 is configured to move radially within the heating element 160 such that the second longitudinal axis C aligns collinearly with each of the prongs of the multi-pronged susceptor at some point during the movement of the consumable-containing package 102 within the heating element 160. In some embodiments, both the consumable-containing package 102 and the heating element 160 may move. For example, the heating element 160 may move linearly along the longitudinal axis of the consumable-containing package 102, and the consumable-containing package 102 can move in an eccentric or radial path to move the susceptor 106 into position relative to the heating element 106, so that all of the consumables are heated sequentially as the user takes individual puffs. Other variations of movement may also be used.

The movement mechanisms described above are merely examples. The mechanism in an X-Y-Z movement scenario could be accomplished using a variety of combinations of motors, linear actuators, gears, belts, cams, solenoids, and the like.

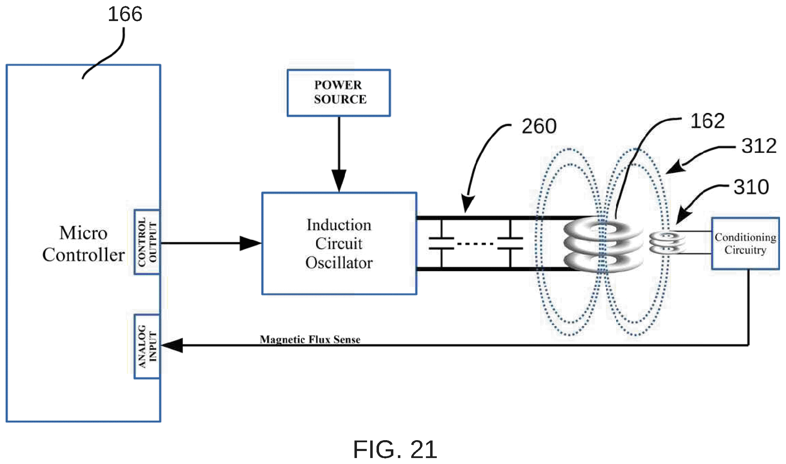

With reference to Figure 21, a closed loop control of the induction heating system can be based on sensing of a magnetic flux density created by the induction heating system. Induction heating systems operate by virtue of creating a concentrated, alternating magnetic field inside of the induction coil heating element. This field will produce a heating effect in a metal susceptor by virtue of the eddy currents and magnetic flux reversal (assuming a ferrous receptor material) that occur in the susceptor material. Induction heating is typically "open loop" in that there are limited means of monitoring of the temperature of the susceptor inside of the induction coil while it is operating. Under controlled conditions, the magnetic field external to the induction coil and in reasonable proximity to the coil can be used determine the intensity of the flux inside of the coil. For example, a small coil 310 can be placed in reasonable proximity to the induction coil-type heating element 160 with its axis approximately parallel to the magnetic flux field lines 312 passing through the small coil 310, providing a means of detection of the magnitude of the magnetic flux of the induction coil-type heating element 160 present by virtue of the voltage induced across the small coil 310 due to the changing magnetic flux passing through the small coil 310. The magnitude of this external flux can then be calibrated to correlate to the magnetic flux density inside of the heating element 160, and therefore, be used as a means of closed loop control of the induction system to ensure consistent performance insofar as heating of the susceptor 106. The magnetic flux is symmetrical around the axis of the induction coil. A measurement of the flux density taken any place near the induction coil can be used to extrapolate the magnetic flux density inside of the heating element, based on characterization of the relative magnitudes of the magnetic flux in each location (inside of the induction coil and inside of the parasitic sensing coil). In practice, there is no need to quantify this, as the flux sensing is instead used to infer the rate of heating that will occur in a susceptor 106 that is present in this magnetic field. Thus, the small coil 310 configured in this way functions as a magnetic flux sensor.

Therefore, in some embodiments, the device may further comprise a magnetic flux sensor adjacent to the inductive heating element 160 and configured to measure a magnetic flux created by the inductive heating element 160. The magnetic flux sensor may be operatively connected to the controller 166 to control activation of the inductive heating element 160 based on feedback from the magnetic flux sensor.

**Heat Sink**

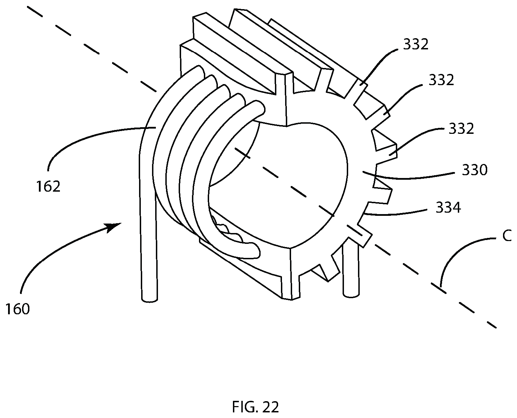

In some embodiments, to manage the thermal heat dissipation from the heating element 160, the device may further comprise a heat sink 330 operatively connected to the inductive heating element 160. Induction heating involves the circulation of high currents in the induction coil, resulting in resistive heating in the wire used to form the coil. Thermal heat dissipation takes advantage of materials with high thermal conductivity that are electrically insulating to form heat sinks 330. Preferably, heat sinks 330 can be formed either through injection molding or potting processes. Because the preferred embodiment utilizes a cylindrical coil as the heating element 160, the heat sink 330 may also be a cylinder formed around the induction coil, so that it encapsulates the coil as shown in Figure 22. The cylindrical heat sink 330 encapsulating the heating element 160 resides within a vertical cavity inside the case 202, forming a sort of "chimney" within which air convection occurs. The chimney requires venting at the top to support the airflow. This method also eliminates fringing of the electromagnetic field, allowing for a very focused heating method on each segment of the consumable-containing package 102. As a result of such focus, it would not be necessary to wrap the consumable-containing unit 104 inside the consumable-containing package 102 in a non-conductive foil or other similar material, paper or a similar material would suffice.

In the preferred embodiment, the heat sink 330 is a finned cylinder encompassing the inductive heating element 160. The finned cylinder is a cylindrically shaped heat sink with fins 332 projecting laterally away from its exterior surface 334. Preferably each fin 332 extends substantially the length of the cylinder to provide a substantial surface area from which heat from the heating element 160 can dissipate. The thermally conductive material of the heat sink 330 may be a polymer. Thermally conductive polymer may be a thermoset, thermoplastic molding or potting compound. The heat sink 330 may be machined, molded or formed from these materials. Material could be rigid or elastomeric. Some examples of the thermally conductive compounds used in thermally conductive polymers are aluminum nitride, boron nitride, carbon, graphite and ceramics. In the preferred embodiment, the heating element 160 is an inductive coil wrapped in a finned cylinder of a thermally conductive polymer that has been molded around the coil, with an open center creating venting via a chimney-like effect.

**Pressure Control**

The extraction of the aerosol from the consumable containing unit 104 is assisted by negative pressure created within the housing 150. There are multiple purposes served by this negative pressure. First, the negative pressure facilitates and accelerates the extraction of the heated and expanding aerosol being produced inside of the consumable containing unit 104 as a result of the induction heating of the susceptor 106 within the encasement 108, and the maximal contact between the susceptor 106 with a mostly plant-based substance, such as tobacco or a cannabinoid.

Second, the negative pressure inside the housing 150 provides a means of detecting when a user is drawing air through the housing 150 through the use of a pressure sensor that is monitored by the system controller 166 integral to the aerosol producing device 200, which is then used to initiate a change in the control of the circuitry used to heat the consumable containing unit 104.

Third, the negative pressure inside the housing 150 provides a means of controlling the amount of aerosol production in order to change the user experience by providing the user controllable or automatic control of the air orifice.

The negative pressure inside the housing 150 can be created simply by the restriction of airflow created by the end cap 154 while the user is drawing in air from the mouthpiece 158.

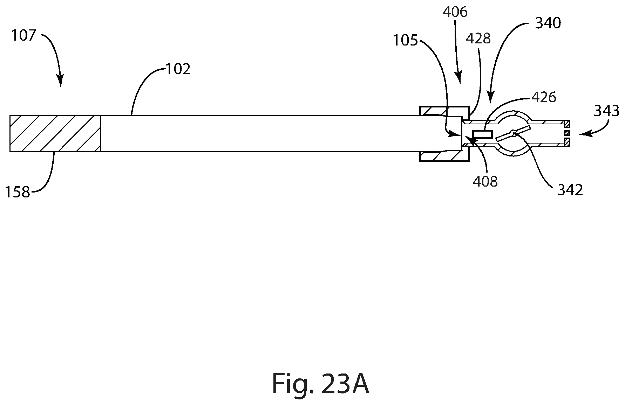

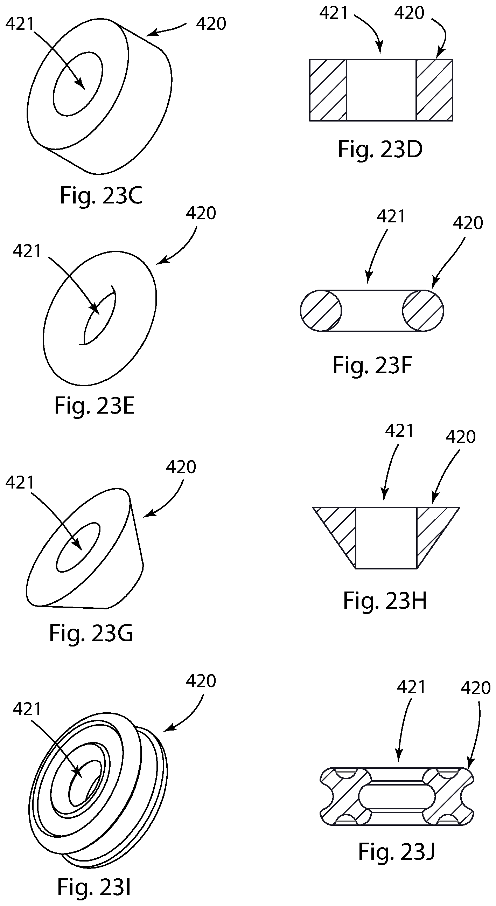

In some embodiments, as shown in Figure 23A, the device may further comprise an airflow controller 340 to provide a means for adjusting the flavor robustness of the consumable-containing unit 104 by controlling the airflow that is drawn through the consumable-containing package 102. The design of the consumable-containing package 102 is such that the amount of vapor/flavor that is introduced into the airflow passageways is a function of the duration and intensity of induction heating, and the air pressure differential between the air passageway(s) through the consumable-containing package 102. For example, the airflow controller 340 may comprise an adjustable flow control valve 342, such as a needle valve, butterfly valve, ball valve, or an adjustable aperture. Adjustable flow control valves allow the user to control the airflow even during use. However, the airflow controller 340 may also be a membrane 344 with fixed apertures, such as a porous or fibrous membrane or element. A membrane 344 may also act as an intake particulate filter. Therefore, flow control mechanisms may or may not be user adjustable. In the membrane 344 embodiments, there may be provided multiple membranes 344 with different sized apertures. Thus, the user can select the desired aperture size and apply that membrane 344 to the first end 105 of the device. If the user prefers increased or decreased airflow, the user can select another membrane 344 with larger or smaller apertures, respectively. In some embodiments, the airflow controller 340 may use both a control valve 342 and a membrane 344. For example, the membrane 344 may be precede the control valve 342 so as to control airflow and filter particulates before the control valve 342, then the control valve 342 can further control the airflow for fine-tuned control of the airflow.

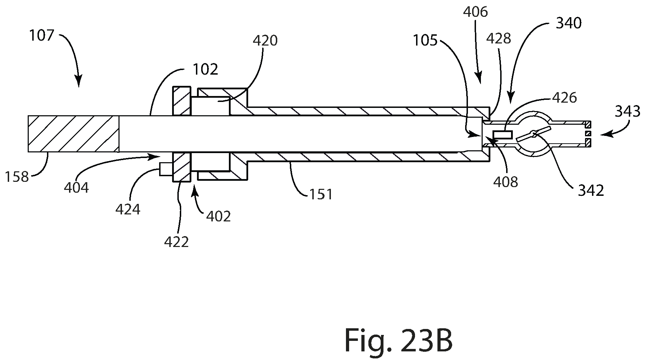

In some embodiments, to better control the efficiency of drawing out the heated consumable aerosol out of the encasement 108, a receiver 151 may be provided as shown in Figure 23B. The receiver 151 has a proximal end 402 with a main opening 404, and a distal end 406 with a distal opening 408. The first end 105 of the consumable-containing package 102 can be inserted through the main opening 404 of the receiver 151 and seated at the distal end 406 adjacent to the distal opening 408. As such, the main opening 404 is substantially similar in size and shape as the outer surface of the consumable-containing package 102 (e.g., the outer dimensions of the housing 150). The distal opening 408 of the receiver 151 is smaller than the main opening 404, and the first and second openings 153, 157 of the housing 150. The smaller size of the distal opening 408 relative to the openings 153, 157 of the housing 150 restricts the airflow into the receiver 151, thereby decreasing the pressure inside the receiver 151 and the housing 150.

Preferably, the receiver 151 would be made of non-conductive material to avoid induction heating, such as borosilicate ceramic, plastic, wood, carbon fiber, glass, quartz glass, Pyroceram glass, Robax glass, high-temperature plastics such as Vespel, Torlon, polyimide, PTFE (polytetrafluoroethylene), PEEK (polyetheretherketone), or other suitable materials. Alternatively, the receiver 151 could be made of a conductive material that has a lower resistivity than the susceptor 106 in the consumable-containing package 102, which would allow some induction heating of the receiver 151, but not as much as the susceptor 106. Examples of lower-resistive materials may include copper, aluminum, and brass, where the susceptor 106 is made of higher-resistance materials such as iron, steel, tin, carbon, or tungsten, although other materials may be used. In some embodiments, a receiver 151 with an equal or higher resistivity than the susceptor 106 may be used, which will heat the outside of the consumable-containing package 102 as the receiver 151 heats up via induction. The receiver 151 can be fixed to the device 200 and aligned properly with the coils 162 wrapped around the receiver 151 such than when the consumable-containing package 102 is inserted into the receiver 151, the susceptor 106 is properly aligned with the coils 162.

In certain methods of inductively heating, but not burning, the consumable contained in the consumable-containing package 102 is inserted into the receiver 151 by the user. In doing so there may be a gap between the consumable-containing package 102 and the receiver 151. Such a gap can be the cause of three significant problems. First, in embodiments with a valve 342, the gap will, in certain circumstances, cause the airflow to come from the top and outside of the receiver 151 to the bottom, causing the valve 342 to operate inefficiently and resulting in an unsatisfactory consumer experience. Second, users experience sticky lips in which their lips stick to the mouthpiece 158, and therefore, accidentally pull the consumable-containing package 102 out from the receiver 151 after inhaling the active ingredient due to the consumable-containing package sticking to the lip of the consumer and there not being a seal to keep the consumable-containing package 102 secure during otherwise normal use. And, third, in certain embodiments of the consumable, where the consumable is a pill shape and compressed around a susceptor 106, the gap between the mouthpiece and the consumable would complicate the proper amount of air allowed into the heating receiver 151.

In some embodiments, a seal 420 can be provided at the proximal end 402 of the receiver 151. The seal 420 can surround the housing 150 to create an airtight seal around the housing 150. For example, the seal 420 can be a ring placed around the housing 150 at the proximal end 402 of the receiver 151. In the preferred embodiment, the seal 420 is a retaining and sealing structure, which can be described as a collet, gasket, O-ring, gland or other similar term that describes a sealing structure. The seal 420 is made from a flexible (elastomeric) polymer such as silicone, rubber, plastic, or other material (thermoset or thermoplastic).

By way of example only, the seal 420 the seal 420 design may be a cylinder with right angle edges and a hole 421 through the center (see, Figure 23C-23D). In some embodiments, the seal 420 may be toroidal or "doughnut shaped" with round edges with a hole 421 through the center (see, Figure 23E-23F). In some embodiments, the seal 420 shape may be conical with angled walls and a hole 421 through its center (see, Figure 23G-23H). In some embodiments, the seal 420 may have multiple contact point sealing surfaces (see, Figure 23I-23J).

Prior to using the device 100, the consumable-containing package 102 is inserted into the main opening 404 of the receiver 151 at the proximal end 402. The seal 420 is positioned upstream of the main opening 404 inside the receiver 151, and surrounding the perimeter or outer surface of the consumable-containing unit 102. In some embodiments, the seal 420 is configured to create a biasing force against the inner wall of the receiver 151 and the outer wall of the consumable-containing package 102, thereby sealing the consumable-containing unit 102 against the receiver 151.

In some embodiments, a presser 422 allows for actuation of the seal 420 from a released state to an active state. The released state allows for free movement of the consumable-containing package 102 into and out of the receiver 151. In the active state, the presser 422 radially compresses the seal 420 against the consumable-containing package 102. For example, in some embodiments, the presser 422 may axially compress the seal. The seal 420 being elastic, when axially compressed, expands radially, thereby reducing its inside diameter such that it compresses and seals around the perimeter wall of the consumable-containing unit 102. This type of compression can be achieved through rotational or linear movement of the presser 422 within the handheld device 100. This compression and contact with the consumable-containing unit 102 can either serve the function of retaining the consumable-containing unit 102 inside the device 100 while in operation, preventing it from being easily dislodged or prematurely removed from the handheld device 100, or it can also serve the function of retaining and providing a seal around the perimeter of the consumable-containing unit 104. As such, the presser 422 can be a mechanical or electromechanical mechanism to compress the seal 420 through actuation of an actuator 424, such as a button, knob, slide, touch screen, switch, dial, and the like, or any combination thereof.

The receiver 151 in combination with the seal 420 restrict the source of all airflow drawn through the consumable-containing package 102 to be sourced via the distal opening 408. This precise control of airflow translates directly to control of the negative pressure generated inside of the consumable-containing package 102. Upon inserting the consumable-containing package 102 into the receiver 151 of the device 100, actuating the seal 420, the user inhales through the exposed mouthpiece 158 (filtered) of the consumable-containing unit 102, and the flow of air is drawn through the distal opening 408, producing a pressure drop at the first end 105 of the consumable-containing package 102 as well as internal to the consumable-containing unit 104. This pressure drop (vacuum) at the first end 105 of the consumable-containing package 012 can be sensed by a pressure sensor 426 operatively connected to the system controller 166 to initiate and control the heating of the medicant or vapor producing components inside of the consumable-containing package 102.

In some embodiments, the seal 420 and the airflow controller 340 can be used together. Preferably, the airflow controller 340 is connected to the consumable-containing package 102 at the first end 105. Distal from the first end 105 of the consumable-containing package 102 is a distal end 345 of the airflow controller 340 having a distal opening 343. In between the distal opening 343 of the airflow controller 340 and the first end 105 of the consumable-containing package 102 is a control valve 342. The control valve 342 can be a needle valve, butterfly valve, ball valve, check valve, or any other adjustable flow valve, or an adjustable aperture. The control valve 342 allows the user to control the airflow even during use. The distal opening 343 can be smaller than the opening 153 at the first end 152 of the housing 150 to provide a restriction in airflow through the first end 152 of the housing 150, providing a controlled pressure drop as a function of airflow by throttling the flow of air through the distal opening 343 or valve 342. The control valve 342 can be operatively connected to the system controller 166 to control 342 the opening and closing of the valve 342.

The airflow controller 340 can be used in place of the end cap 154 or to supplement the end cap 154. If the airflow controller 340 is used with the end cap 154, the airflow controller can be placed adjacent to the end cap 154 on the housing 150. In some embodiments, rather than having the end cap 154 on the housing 150, the end cap 154 may be placed upstream or down stream of the control valve 342 within the airflow controller 340. With or without the end cap 154, the airflow controller 340 provides a means for controlling the airflow that is drawn upstream to downstream through the consumable-containing package 102, and the vacuum or air pressure differential created inside the housing 150. This pressure differential draws the vapor out of the consumable-containing package 102 and into the airflow. If the airflow into the first end 105 of the consumable-containing package 102 can be controlled, this pressure differential can be varied, allowing more (or less) vapor to be introduced into the airflow, effectively altering the robustness of the flavor. This ability to alter the flavor robustness is closely integrated with the heating of the consumable-containing package 102, as it is the rise in temperature of the consumable that produces this vapor. By precise control of the heating process (time and rate) and the airflow through the first end 105 of the consumable-containing package 102, wide range of flavor robustness experiences can be produced.

In some embodiments, the airflow controller 340 can be a part of the receiver 151. For example, the airflow controller 340 can be attached to or integrally formed with the distal end 406 of the receiver 151. As such, the airflow controller 340 creates an extension of the receiver 151. To prevent the first end 152 of the housing 150 from falling into the control valve 342, a stop 428 can be placed towards the distal end 406 of the receiver 151, but downstream of the control valve 342. For example, the stop 428 can be a projection, wall, protrusion, or the like, projecting radially inwardly from the inner wall to abut against the first end 152 of the housing 150 and prevent the housing from moving into the air controller 340.

The control valve 342 serves as an airflow control into the first end 152 of the housing 150 when the user is drawing air through the consumable-containing package 102. By restricting this airflow a negative pressure (vacuum) is developed at the first end 152 of the housing 150 as well as internal to the housing 150. In the preferred embodiment, the airflow through this valve 342 could be controlled via either a mechanical or electromechanical means integral to the device 100. The action of the control valve 342, pressure sensor 426, and the seal 420 can be coordinated by the system control 166 for managing the precise pressure differential built up inside the consumable-containing package 102.

Using the seal 420 with the control valve 342 makes the control valve 342 work better as it would allow for better control of the control valve 342 as the seal 420 makes sure that air flow cannot come from the top at the mouthpiece 158, or any other areas that air can leak into the device 100 other than the openings 408, 343 specifically designed for airflow. So, functioning of the valve 342 is enhanced by the seal 420.

The seal 420 also provides another advantage. When using devices such as the present invention, there can a tendency for the lips to stick to the mouthpiece. When this happens, the sticky lips can pull the consumable-containing package 102 out from the aerosol producing device 200. This phenomenon does not occur in regular cigarettes because the unitary construction; however, for a device where the consumable-containing package 102 is separate from104 the aerosol producing device 200, the aerosol producing device 200 has to hold the consumable-containing package 102. The seal 420 of the present invention makes it impossible for sticky lips to pull the consumable-containing package out from the aerosol producing device 200, unless doing so is specifically desired.

In embodiments utilizing the receiver 151, the housing 150 becomes optional because the receiver 151 essentially functions as the housing 150. Therefore, the consumable-containing unit 104 can be inserted directly into the receiver 151 in which case the receiver 151 is the housing 150. The mouthpiece 158 can be inserted and sealed at the main opening 404 of the receiver 151 with the seal 420. In such a case the seal 420 between the receiver 151 and the mouthpiece 158 would be critical to its operation and the only air allowed would be what enters through the distal opening 408 or the valve 342.

Therefore, in some embodiments a device 100 for generating aerosol, comprises a consumable-containing unit 104; a susceptor 106 combined with the consumable-containing unit 104; a receiver 151 having a proximal end 402 defining a main opening 404 to receive the consumable-containing unit 104, and distal end 406 defining a distal opening 408; a mouthpiece 158 inserted into the main opening 404 of the receiver 151; and a seal 420 to create an airtight seal between the mouthpiece 158 and the main opening of 404 the receiver 151. The device 100 may further comprise an encasement 108 encasing the consumable-containing unit 104 and the susceptor 106 to form a consumable-containing package 102. The device 100 may further comprise a housing 150 to receive the consumable-containing package 102. In such an embodiment, the mouthpiece 158 is incorporated into the housing 150. The device 100 can further comprise a spacer 135 in between the consumable-containing package 102 and the housing 150 to maintain an airflow passageway to the mouthpiece 158. To control the pressure differential inside the receiver 151 relative to outside the receiver 151, the device 100 may further comprise an airflow controller 340 at the distal end 406 of the receiver 151. The airflow controller 340 comprises a control valve 342 and a pressure sensor 426. The pressure sensor 426 is operatively connected to a system controller 166, and the system controller 166 is operatively connected to the control valve 342 so as to control the pressure differential inside the receiver 151 relative to the outside of the receiver 151 to optimize the aerosolization and airflow of the consumable.

**Hollow Susceptors**

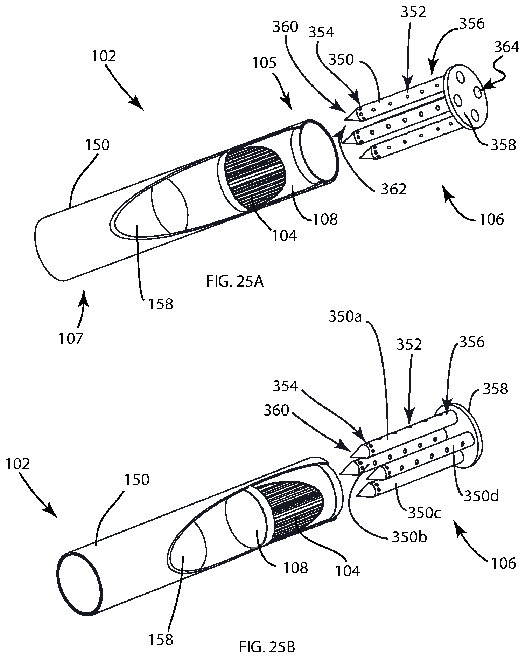

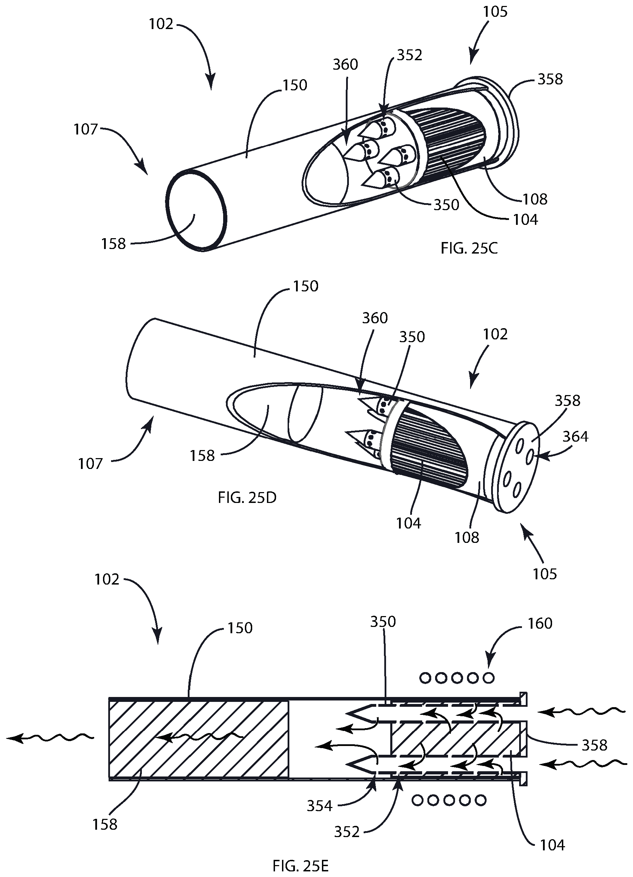

In some embodiments, rather than having the aerosol flow from the consumable-containing unit 104 through openings 120 of the encasement 108 into a filter tube 140, and towards the mouthpiece 158, the air flows into the susceptor 106, draws out the active from the consumable-containing unit 104 to create the aerosol that flows through the susceptor 106 towards the mouthpiece 158, as shown in Figure 25A-E. In such, embodiments, the susceptor 106 may have one or more hollow prongs 350 with at least one inlet 352 along the length of the each prong 350, and at least one outlet 354. The prong 350 comprises a connected end 356 operatively connected to a susceptor base 358, and a free end 360 opposite the susceptor base 358. The hollow prong 350 is connected to the susceptor base 358 at the connected end 356. The outlet 354 of the hollow prong 350 is located towards the free end 360. For example, the outlet may be at the tip 362 of the free end 360, or there may be a plurality of outlets 354 angularly spaced apart around the perimeter surface of the hollow prong 350 at the free end 360 side.

In some embodiments, the tip 362 of the free end 360 may be pointed or sharp to facilitate penetration into the consumable-containing unit 104. The particle size, density, binders, fillers or any component used in the consumable-containing unit 104 may be engineered to allow the penetration of the susceptor prongs 290, 350 and/or perforation needles without causing excessive compression or changes to the density of consumable-containing unit 104. Changes to the density from compression "packing" of consumable containing unit 104 could negatively effect air or vapor flow through the consumable-containing unit 104.

Any consumable particulate that may be pushed thorough the encasement 108 after susceptor 106 penetration would be held captive in the cavity 368 between consumable-containing unit 104 and mouthpiece 158. Since tips 362 of the prongs 290, 350 are sharp it is unlikely that consumable will be ejected out from the encasement 108.

In some embodiments, the outlets 354 and/or the inlets 352 may be covered with the coating that melts away at heated temperatures. In the preferred embodiment, the consumable-containing unit 104 is long enough to cover the entire hollow prong 350 except for the outlet 354.

The susceptor base 358 may comprise an opening 364 that corresponds with the hollow prong 350. In embodiments with multiple hollow prongs 350a-d, each hollow prong 350a-d has its own corresponding opening 364.

In some embodiments, there may be multiple hollow prongs 350a-d. The hollow prongs 350a-d may be arranged in a circle making it compatible with the moving heating element 160 or moving consumable-containing package 102. In some embodiments, there may be a single hollow prong 350 with the hollow prong 350 centered in the susceptor base 358. In some embodiments, there may be a center hollow prong 350 surrounded by a plurality of hollow prongs 350a-d. Other hollow prong 350 arrangement can be used.

Each hollow prong 350 may have at least one inlet 352 and at least one outlet 354. Preferably, the hollow prong 350 comprises a plurality of inlets 352 and a plurality of outlets 354. The inlets 352 may be arranged in a series along the length of the hollow prong 350. In some embodiments, the inlets 352 may be circularly arranged about the perimeter of the hollow prong 350. Increasing the number of inlets 352 on a hollow prong 350 increases the number of points through which the aerosol generated can escape from the consumable-containing unit 104 and out of the consumable-containing package 102. Similarly, there may be a plurality of outlets 354 circularly arranged about the perimeter of a prong 350 at the free end 360 side.

In some embodiments, the consumable-containing unit 104 does not extend from one end 105 of the consumable-containing package 102 to the mouthpiece 158. As such, a cavity 368 exists in between the consumable-containing unit 104 and the mouthpiece 158. This cavity 368 can be filled with thermally conductive material, flavoring, and the like.

As shown in the cross-sectional view of Figure 25E, in use, the susceptor 106 is embedded in the consumable-containing unit 104. When the susceptor 106 is heated via inductive heating by the heating element 160, the consumable-containing unit releases the aerosol. As the user sucks on the mouthpiece 158, the pressure differential inside the consumable-containing package 102 causes the aerosol to enter into the hollow prong 350 through the inlet 352 and exit through the outlet 354 (see arrows showing airflow). The aerosol then enters the cavity 368 of the consumable-containing package 102 and is filtered through the mouthpiece 158 for inhalation by the user. As such, the encasement 108 need not have any openings 120.

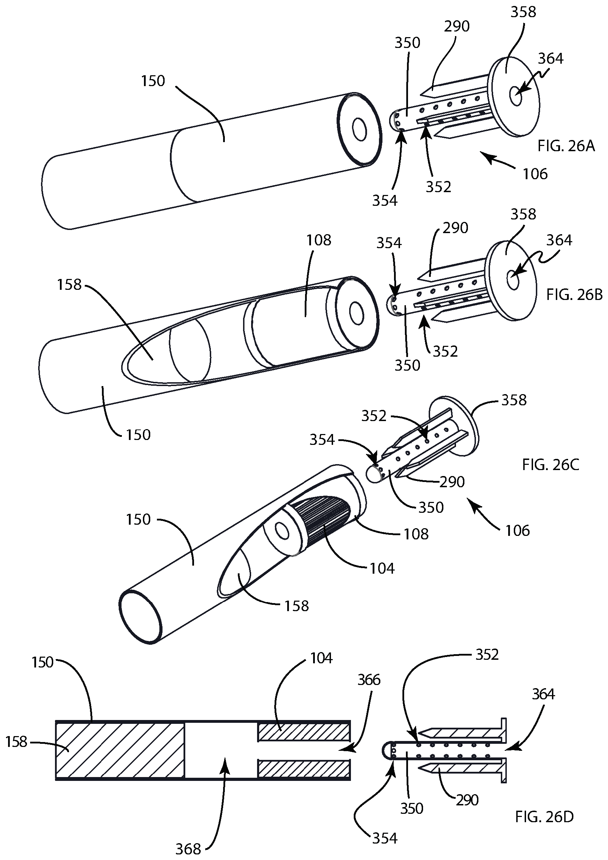

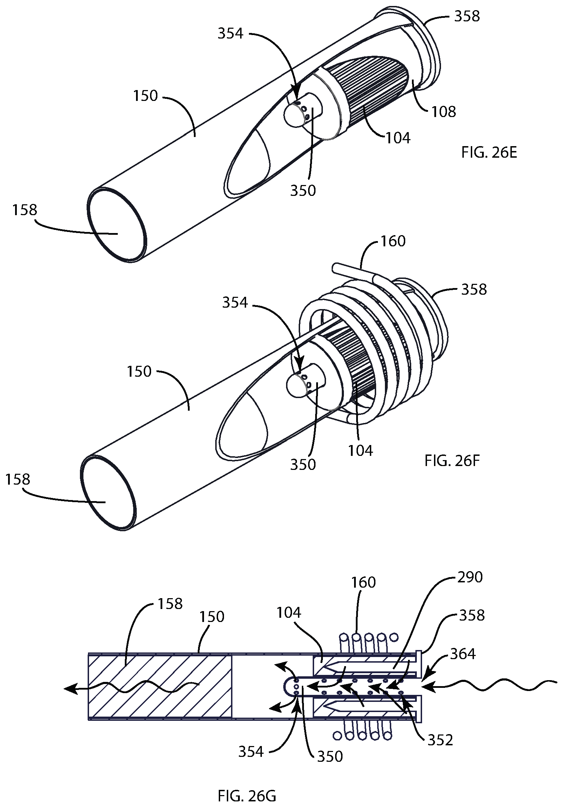

In some embodiments, as shown in Figures 26A-G, there may be a single hollow prong 350 centrally positioned on the susceptor base 358, with a plurality of prongs 290a-d surrounding the hollow prong 350. In such an embodiment, the hollow prong 350 need not be capable of heating via induction heating, although it can be. In this embodiment, the consumable-containing unit 104 may have a central hole 366 through which the hollow prong 350 can be inserted for a tight fit.

As shown in Figure 26G, in use, when the susceptor prongs 290 are heated, the aerosol generated enters through the inlets 352 of the hollow prong 350 and exits through the outlets 354 and into the mouthpiece 158 as shown by the airflow arrows.

Aerosol produced by the methods and devices described herein is efficient and reduces the amount of toxic byproducts seen in traditional cigarettes and other heat-not-burn devices.

**EXAMPLE**

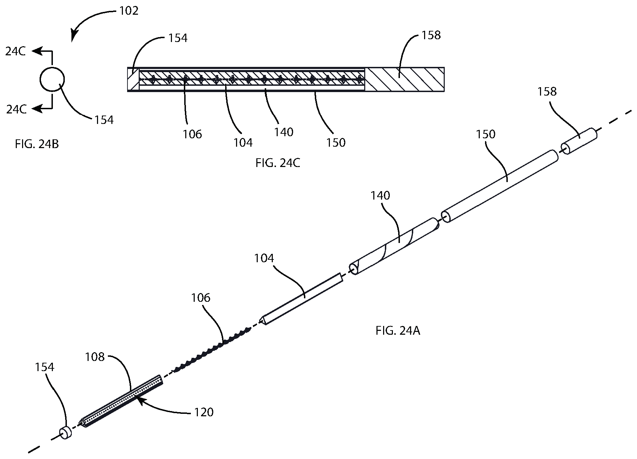

As shown in Figures 24A-C, testing was conducted on consumable-containing packages 102 that were prepared by compressing powdered tobacco mixed with an humectant and PGA, to form the consumable unit 104, around a susceptor 106, encased in a foil covering as the encasement 108, inserted into a filter tube 140 in such a way that openings 120 were present on three sides as air channels, covered in standard cigarette paper as the housing 150, capped on one end with a high flow proximal filter as the mouthpiece 158 and on the other end with a distal filter tip as the end cap 154. The susceptor 106 is in the form of a metal sheet twisted into a spiral. The consumable-containing unit 104 and the encasement 108 have triangular cross-sections. The filter tube 140 is a spiral paper tube.

The testing in Durham, North Carolina was done with a prototype device that was determined to have heated the susceptor to 611C (Degrees Centigrade) by virtue of calibrating the electrical power that was used in the testing process.

The Durham test was conducted using a SM459 20-port linear analytical smoking machine and was performed by technicians familiar with the equipment and all associated accessories. Technicians placed three consumable-containing packages 102 in the smoking machine. Each consumable-containing package 102 was then "puffed" 6 times for a total of 18 puffs. The resulting aerosol was then collected on filter pads. The "smoking" regimen was a puff every 30 seconds with 2-second puff duration and a volume of 55 mL collected using a bell curve profile. The analysis of the collected aerosol determined that 0.570 mg of carbon monoxide (CO) was present in the aerosol of each consumable stick, well below the levels at which it could be assumed that combustion has occurred, despite the fact that it is generally assumed that combustion will occur at temperatures greater than 350C.

A second set of tests was conducted in Richmond, Virginia. The Richmond tests were done with a similarly configured consumable-containing package 102 and a prototype device that was calibrated to heat a susceptor 106 at three separate settings of 275C, 350C and 425C. CO data was generated by Enthalpy Analytical (EA) (Richmond, Virginia, USA), LLC in accordance with EA Method AM-007. Consumable-containing packages 102 were smoked using an analytical smoking machine following the established, Canadian Intense smoking procedure. The vapor phase of the smoke (i.e. aerosol) was collected in gas sampling bags attached to the smoking machine configured to the requested puffing parameters. A non-dispersive infrared absorption method (NDIR) is used to measure the CO concentration in the vapor phase in percent by volume (percent vol). Using the number of consumable-containing packages 102, the puff count, the puff volume, and ambient conditions, the percent CO was converted to milligrams per consumable-containing package (mg/cig).

At the calibrated temperature settings it was determined that no CO was found to be in the aerosol produced at each of the settings, despite the fact that it is generally assumed that combustion will occur at temperatures greater than 350C.

The tests conducted are industry standard tests. In similar industry standard tests, commercially available heat-not-burn products report CO at 0.436 mg/cig. Standard combustible cigarette reports CO at 30.2 mg/cig.

The foregoing description of the preferred embodiment of the invention has been presented for the purposes of illustration and description. It is not intended to be exhaustive or to limit the invention to the precise form disclosed. Many modifications and variations are possible in light of the above teaching. It is intended that the scope of the invention not be limited by this detailed description, but by the claims and the equivalents to the claims appended hereto.

**Claims**

1. A method for aerosolizing a consumable by an aerosol producing device, the method comprising:

    a) preheating a susceptor surrounded by the consumable to a moderate temperature that is above room temperature, but below an aerosolizing temperature; and
    b) heating the susceptor from the moderate temperature to the aerosolizing temperature, whereby the consumable is aerosolized.

2. The method of claim 1, further comprising calculating an amount of power needed to heat the susceptor to the aerosolizing temperature by extrapolating a temperature drop over time.

3. The method of any one of claims 1 to 2, wherein heating the susceptor to the aerosolizing temperature occurs upon when a pressure sensor detects a pressure drop inside the aerosol producing device.

4. The method of any one of claims 1 to 3, wherein preheating the susceptor occurs upon insertion of a consumable-containing package into the aerosol producing device.

5. The method of any one of claims 1 to 3, wherein preheating the susceptor is initiated by actuation of an external actuator.

6. The method of any one of claims 1 to 5, wherein after heating the susceptor to the aerosolizing temperature, bringing the susceptor back down to the moderate temperature.

7. The method of any one of claims 1 to 5, wherein after heating the susceptor to the aerosolizing temperature, bringing the susceptor back down to an intermediate temperature that is lower than the aerosolizing temperature, but higher than the moderate temperature.

8. The method of any one of the preceding claims, wherein the moderate temperature is in a range from about 100 degrees Celsius to about 250 degrees Celsius.

9. The method of any one of the preceding claims, wherein the aerosolizing temperature is approximately 350 degrees Celsius or higher.

10. The method of any one of the preceding claims, wherein the preheating and the heating are performed by a heating element.

11. The method of claim 10, wherein the heating element is a coil wrapped around the susceptor.

12. The method of any one of the preceding claims, further comprising measuring the moderate temperature with a temperature sensor operatively connected to a system controller.

Fig. 1

Fig. 2A

Fig. 2B

EP 4 776 446 A2

Fig. 2C

Fig. 2D

EP 4 776 446 A2

Fig. 2E

Fig. 2F

Fig. 3A

Fig. 3B

Fig. 3C

Fig. 3D

Fig. 4A

**Fig. 4B**

EP 4 776 446 A2

Fig. 4C

Fig. 4D

Fig. 4E

Fig. 5A

Fig. 5B

Fig. 5C

Fig. 6A

Fig. 6B

108

120

104

Fig.7A

108a

108a

104

120

Fig.7B

**Fig. 7C**

**Fig. 7D**

Fig. 8A

Fig. 8B

Fig. 9A

Fig. 9B

Fig. 9C

FIG. 10A

FIG. 10B

EP 4 776 446 A2

Fig. 11A

Fig. 11B

Fig. 11C

Fig. 11D

Fig. 11E

105

150

FIG. 12A

281

280

150

FIG. 12B

105

150

281

FIG. 12C

280

105

M

281

280

105

150

FIG. 12D

280

FIG. 12E

FIG. 13A

FIG. 13B

160

106

162

290a

102

290b

290c

FIG. 13C

106

162

102

160

FIG. 13D

FIG. 14A

FIG. 14B

FIG. 14C

FIG. 15A

FIG. 15B

FIG. 15C

FIG. 16A

FIG. 16B

FIG. 16C

FIG. 16D

302

300b

300a

162   106

160

FIG. 17A

102

302

300b

162

102

300a

160   FIG. 17B

FIG. 18A

FIG. 18B

FIG. 19

FIG. 20

FIG. 21

FIG. 22

Fig. 23A

Fig. 23B

Fig. 23C

Fig. 23D

Fig. 23E

Fig. 23F

Fig. 23G

Fig. 23H

Fig. 23I

Fig. 23J

FIG. 24B

FIG. 24C

FIG. 24A

FIG. 25A

FIG. 25B

FIG. 25C

FIG. 25D

FIG. 25E

FIG. 26A

FIG. 26B

FIG. 26C

FIG. 26D

358

354

150

158

104

108

350

FIG. 26E

160

354

358

150

158

104

350

FIG. 26F

158  150

160

290

104

358

364

354

350

352

FIG. 26G

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63000456